# EUROPÄISCHE PATENTSCHRIFT

(11) **EP 0 939 765 B1**
(45) Veröffentlichungstag und Bekanntmachung des Hinweises auf die Patenterteilung: **05.12.2001**
(21) Anmeldenummer: 97910297.7
(22) Anmeldetag: 17.09.1997
(51) Int. Cl.: C07K 5/068, A61K 38/05, A61K 47/48

(54) **GLYCOKONJUGATE VON MODIFIZIERTEN CAMPTOTHECIN-DERIVATEN(A- ODER B-RING-VERKNÜPFUNG)**
GLYCOCONJUGATES OF MODIFIED CAMPTOTHECIN DERIVATIVES (A- OR B-RING LINKAGE)
COMPOSES GLYCO-CONJUGUES DE DERIVES DE CAMPTOTHECINE MODIFIES (LIAISON A UN CYCLE A OU B)

(30) Priorität: 30.09.1996 DE 19640207
(43) Veröffentlichungstag der Anmeldung: 08.09.1999
(73) Patentinhaber: BAYER AG, 51368 Leverkusen (DE)
(72) Erfinder: LERCHEN, Hans-Georg, D-51375 Leverkusen (DE); VON DEM BRUCH, Karsten, D-51375 Leverkusen (DE); BAUMGARTEN, Jörg, D-42115 Wuppertal (DE); SPERZEL, Michael, D-42275 Wuppertal (DE)
(86) Internationale Anmeldenummer: EP9705089
(87) Internationale Veröffentlichungsnummer: WO9814468

(56) Entgegenhaltungen:
- EP-A- 0 418 099
- EP-A- 0 624 377
- EP-A- 0 640 622
- EP-A- 0 757 049
- EP-A- 0 781 781
- DE-A- 4 236 237

## Beschreibung

Die vorliegende Erfindung betrifft Glycokonjugate von Camptothecin-Derivaten, in denen mindestens eine Kohlenhydrat-Komponente über geeignete Spacer und Abstandhalter mit dem A- oder B-Ring eines Camptothecin-Derivats verknüpft ist. Die Erfindung betrifft weiterhin Verfahren zur Herstellung der erfindungsgemäßen Verbindungen sowie deren Verwendung als Arzneimittel, insbesondere im Zusammenhang mit Krebserkrankungen.

20(S)-Camptothecin ist ein pentacyclisches Alkaloid, das 1966 von Wall et al. isoliert wurde (J.Am.Chem.Soc. 88, 3888 (1966)). Es besitzt ein hohes Antitumor-Wirkpotential in zahlreichen In-vitro- und In-vivo-Tests. Leider scheiterte jedoch die Realisierung des vielversprechenden Potentials in der Klinik an Toxizitäts- und Löslichkeitsproblemen.

Durch Öffnung des E-Ring-Lactons und Bildung des Natriumsalzes wurde eine wasserlösliche Verbindung erhalten, die in einem pH-abhängigen Gleichgewicht mit der ringgeschlossenen Form steht. Klinische Studien führten auch hier bisher nicht zum Erfolg. 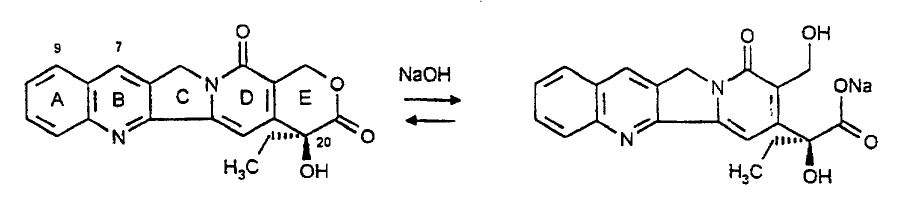 Etwa 20 Jahre später wurde gefunden, daß die biologische Aktivität auf eine Enzyminhibition der Topoisomerase I zurückzuführen ist. Seither wurden die Forschungsaktivitäten wieder verstärkt, um verträglichere und in-vivo wirksame Camptothecin-Derivate zu finden.

Zur Verbesserung der Wasserlöslichkeit wurden Salze von A-Ring- und B-Ring-modifizierten Camptothecin-Derivaten sowie von 20-O-Acyl-Derivaten mit ionisierbaren Gruppen beschrieben (Vishnuvajjala et al. US 4943579). Letzteres Prodrug-Konzept wurde später auch auf modifizierte Camptothecin-Derivate übertragen (Wani et al. WO 9602546). Die beschriebenen 20-O-Acyl-Prodrugs haben allerdings in-vivo eine sehr kurze Halbwertszeit und werden sehr schnell zum Grundkörper gespalten.

Wir fanden nun überraschend, daß die Anknüpfung von Kohlenhydratderivaten über geeignete Spacer an Amino- oder Hydroxyl-Gruppen, die direkt oder über einen Abstandhalter mit dem A- oder B-Ring von Camptothecin-Derivaten verbunden sind, zu einer Verbindungsklasse mit hochinteressanten Eigenschaften führt:
- Die so erhaltenen Konjugate zeigen in-vitro eine hohe Aktivität gegenüber Tumorzellinien und Tumorxenografts.
- Sie weisen gegenüber den zugrundeliegenden Toxophoren eine deutlich höhere Verträglichkeit und Tumorselektivität sowie eine verbesserte Löslichkeit, insbesondere in wäßrigen Medien auf.
- In-vivo zeigen sie exzellente therapeutische Wirksamkeit über mehrere Dosisstufen.
- Sie sind in extrazellularem Medium und in Blut wesentlich stabiler als die in der Literatur beschriebenen 20-O-Acyl-Prodrugs von Camptothecin.

Dokument EP-A-418 099 offenbart 10,11-methylenedioxy-Camptothecin-Derivate, die unter anderem einem zusätzlichen Peptidrest tragen können.
Laut EP-A-418 099 sind die dort offenbarten Verbindungen wasserlöslich und besitzen eine hohe cytotoxische Aktivität in vitro. Es wird allerdings in EP-A-418 099 nicht offenbart, daß die beschriebenen Verbindungen ebenso eine excellente therapeutische Wirksamkeit zeigen, in extrazellulärem Medien und in Blut sehr stabil sind sowie eine deutlich höhere Verträglichkeit und Tumorselektivität aufweisen.

Dokument EP-A-0 640 622 offenbart im allgemeinem Polysaccharidderivate von Wirkstoffen und die erhöhte Tumorselektivität dieser Verbindungen. Camptothecin ist in EP-A-0 640 622 als möglicher Wirkstoff an keiner Stelle erwähnt.

In Dokument DE-A-42 36 237 sind Substrat-Spacer-Wirkstoff Verbindungen und ihre Verwendung als Arzneimittel beschrieben, wobei der Substrat ein enzymatisch abspaltbares Kohlenhydrat ist. Jedoch enthält DE-A-42 36 237 kein Beispiel für eine entsprechende Campthotecin-Verbindung.

Die vorliegende Erfindung betrifft Verbindungen der allgemeinen Formel (I) 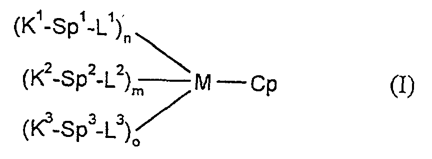 worin
- n, m und o: jeweils für die Zahl 0 oder 1 stehen und n + m + o ≥1 gilt,
wobei
- Cp: für ein Camptothecin-Derivat der Formeln 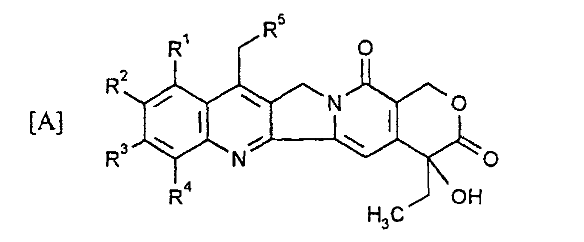 worin
R¹, R², R³ und R⁴ unabhängig voneinander Wasserstoff, Alkyl mit bis zu 3 Kohlenstoffatomen, Halogen, Amino, Hydroxy oder Nitro bedeuten können oder
R² und R³ zusammen eine Gruppe der Formel 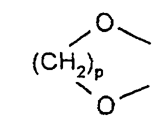 bedeuten, wobei
p die Werte 1 oder 2 annehmen kann, und
R⁵ für -O-* , -NH* oder 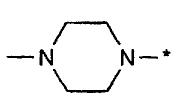 steht, oder für -*Nk⁶ steht, worin R⁶ Arylmethyl oder Hetarylmethyl bedeutet, 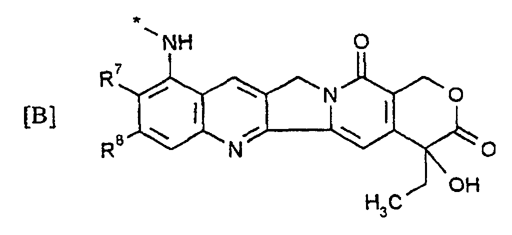 worin
R⁷ und R⁸ wie R² und R³ definiert sind und mit diesen gleich oder verschieden sein können, 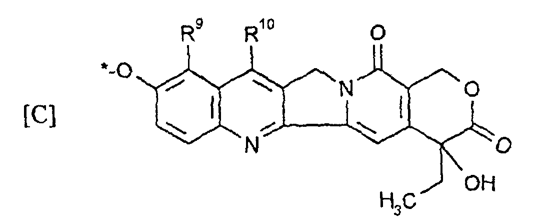 worin
R⁹ für Wasserstoff oder -CH₂-N(CH₃)₂ steht und
R¹⁰ für Wasserstoff oder Ethyl steht, 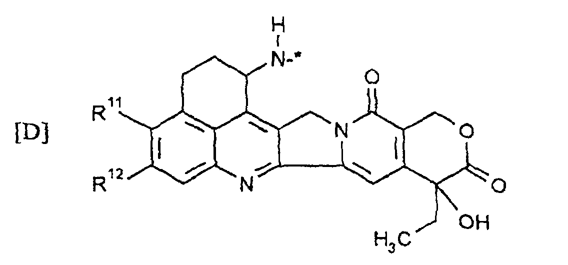 worin
R¹¹ und R¹² wie R² und R³ definiert sind und mit diesen gleich oder verschieden sein können, oder 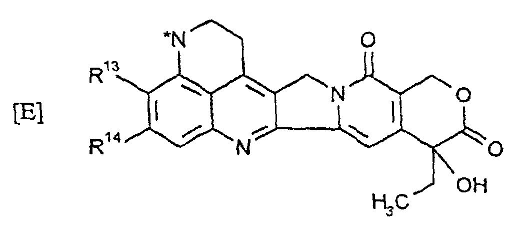 worin
R¹³ und R¹⁴ wie R² und R³ definiert sind und mit diesen gleich oder verschieden sein können, steht, wobei Cp über die mit * markierten Bindungen mit M verknüpft ist,
- M: für eine Brücken-Gruppierung steht, deren Hauptkette bis zu 21 Atome in linearer Abfolge umfaßt,
- L¹, L², L³: unabhängig voneinander für Linker-Gruppierungen stehen, wie sie in der Glycokonjugat-Chemie gängigerweise eingesetzt werden (s. Übersichtsartikel Lee Y.C. and Lee, R. in Lectins and Cancer 1991, 53-69; edited by Gabius M.J. and Gabius S., Springer Verlag),
- Sp¹, Sp² und Sp³: unabhängig voneinander für Arylen mit bis zu 10 Kohlenstoffatomen oder für Alkylen mit bis zu 8 Kohlenstoffatomen stehen die jeweils gegebenenfalls substituiert sind, und
- K¹, K² und K³: unabhängig voneinander für einen Rest der Formel (II) 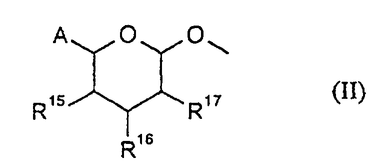 stehen, worin
A Methyl, Hydroxymethyl, Alkoxymethyl mit bis zu 6 Kohlenstoffatomen, Acyloxymethyl mit bis zu 6 Kohlenstoffatomen oder einen Rest der Formel -CH₂-B bedeutet, worin
B für einen Rest der Formel (II) steht,
R¹⁵, R¹⁶ und R¹⁷ unabhängig voneinander Wasserstoff, Hydroxy, gegebenenfalls durch Hydroxy substituiertes Alkoxy mit bis zu 6 Kohlenstoffatomen, gegebenenfalls durch Alkyl oder Acyl mit bis zu 6 Kohlenstoffatomen substituiertes Amino, Halogen, Sulfat oder eine Gruppe der Formeln 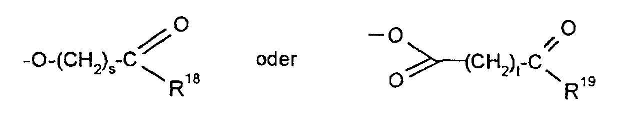 bedeuten, worin
R¹⁸ und R¹⁹ unabhängig voneinander für Hydroxyl oder Alkoxy mit bis zu 6 Kohlenstoffatomen stehen oder für Amino, das gegebenenfalls durch Alkyl mit bis zu 6 Kohlenstoffatomen substituiert ist, stehen und
s und t unabhängig voneinander die Werte 0, 1, 2, 3 oder 4, insbesondere die Werte 1, 2, 3 oder 4 annehmen können oder
R¹⁵, R¹⁶ und R¹⁷ unabhängig voneinander für einen Rest der Formel (II) stehen oder
zwei der Reste R¹⁵, R¹⁶, R¹⁷ zusammen für eine Epoxygruppe stehen,
sowie deren Isomere, Isomerengemische und Salze.

Der Begriff "Alkylgruppen" soll, sofern nicht anders angegeben, im Sinne der Erfindung geradkettige, verzweigte, cyclische und Cycloalkylreste enthaltende Alkylreste umfassen. Diese Definition soll sinngemäß auch für alle anderen Alkylgruppen enthaltenden Reste gelten, wie beispielsweise Alkoxy, Acyl usw..

Die bei der Definition von R⁶ angegebenen Begriffe Arylmethyl und Hetarylmethyl können beispielsweise Phenylmethyl bzw. Pyridylmethyl bedeuten.

Bevorzugt sind Verbindungen der allgemeinen Formel (I), worin K¹, K² und K³ unabhängig voneinander für einen Rest der Formel (II) stehen können, wobei
- A: Methyl, Hydroxymethyl, Methoxymethyl oder Acetoxymethyl bedeutet,
- R¹⁵: Wasserstoff, Hydroxyl, Methoxy oder eine Gruppe der Formeln 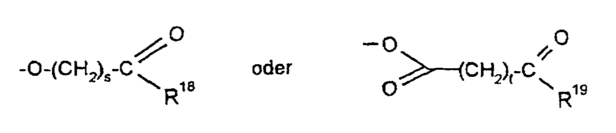 bedeutet, worin
s und t unabhängig voneinander die Werte 1 oder 2 annehmen können und
R¹⁸ und R¹⁹ unabhängig voneinander für Hydroxyl oder Alkoxy mit bis zu 4 Kohlenstoffatomen stehen, oder
- R¹⁵: für einen Rest der Formel (II) steht,
- R¹⁶: Wasserstoff, Hydroxyl, Halogen, Alkoxy mit bis zu 4 Kohlenstoffatomen, Sulfat oder eine Gruppe der Formeln 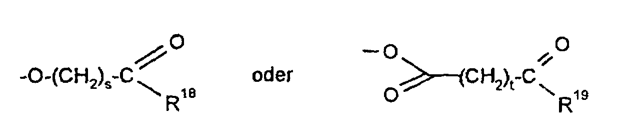 bedeutet, worin
s und t unabhängig voneinander die Werte 1 oder 2 annehmen können und
R¹⁸ und R¹⁹ unabhängig voneinander für Hydroxyl oder Alkoxy mit bis zu 4 Kohlenstoffatomen stehen oder für Amino, das gegebenenfalls durch Alkyl mit bis zu 4 Kohlenstoffatomen substituiert ist, stehen,
- R¹⁷: Hydroxyl, Alkoxy mit bis zu 4 Kohlenstoffatomen, das gegebenenfalls durch Hydroxy substituiert ist, Amino, das gegebenenfalls durch Alkyl oder Acyl mit bis zu 4 Kohlenstoffatomen substituiert ist, oder eine Gruppe der Formeln 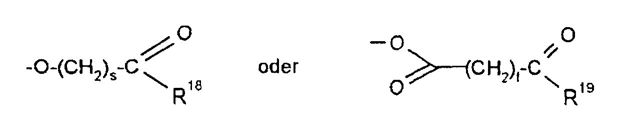 bedeutet, worin
s und t unabhängig voneinander die Werte 1 oder 2 annehmen können und
R¹⁸ und R¹⁹ unabhängig voneinander für Hydroxyl oder Alkoxy mit bis zu 4 Kohlenstoffatomen stehen, oder worin
- R¹⁵ und R¹⁶: gemeinsam für eine Epoxygruppe stehen,
sowie deren Isomere, Isomerengemische und Salze.

Ganz besonders bevorzugt stehen K¹, K² und K³ unabhängig voneinander für einen Rest der Formel (II), wobei
- A: Methyl, Hydroxymethyl, Methoxymethyl oder Acetoxymethyl bedeutet,
- R¹⁵ und R¹⁷: jeweils eine Hydroxylgruppe bedeuten,
und
- R¹⁶: Wasserstoff, Hydroxyl, Halogen, Alkoxy mit bis zu 4 Kohlenstoffatomen, Sulfat oder eine Gruppe der Formeln 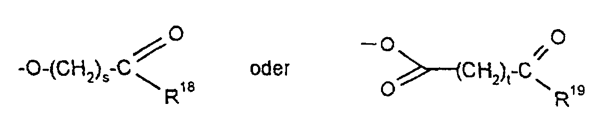 bedeutet, worin
s und t unabhängig voneinander die Werte 1 oder 2 annehmen können und
R¹⁸ und R¹⁹ unabhängig voneinander für Hydroxyl oder Alkoxy mit bis zu 4 Kohlenstoffatomen stehen oder für Amino, das gegebenenfalls durch Alkyl mit bis zu 4 Kohlenstoffatomen substituiert ist, stehen.

Gemäß einer besonders bevorzugten Ausführungsform umfassen die KohlenhydratBausteine K¹, K² und/oder K³ jeweils maximal zwei Monosaccharid-Bausteine.

Weiterhin bevorzugt sind Verbindungen der allgemeinen Formel (I), worin Sp¹, Sp² und/oder Sp³ unabhängig voneinander für Arylen mit bis zu 10 Kohlenstoffatomen stehen können, das mit je einer Gruppe K¹ bzw. K² oder K³ und L¹ bzw. L² oder L³ verknüpft ist und das gegebenenfalls noch ein- oder mehrfach durch Hydroxy, Carboxy, Carboxyalkyl mit bis zu 4 Kohlenstoffatomen, Nitro, Cyano, Halogen, Alkyl mit bis zu 4 Kohlenstoffatomen, Halogenalkyl mit bis zu 4 Kohlenstoffatomen oder durch Alkoxy mit bis zu 4 Kohlenstoffatomen substituiert ist, sowie deren Isomere, Isomerengemische und Salze.

Besonders bevorzugt sind Sp¹, Sp² und/oder Sp³, abgesehen von K¹, K² oder K³ und L¹, L² oder L³, unsubstituiert oder gegebenenfalls substituiert durch Halogen, Nitro, Alkyl mit bis zu 4 Kohlenstoffatomen, Alkoxy mit bis zu 2 Kohlenstoffatomen, -OCF₃ und/oder -CF₃.

Ganz besonders bevorzugt sind Sp¹, Sp² und/oder Sp³ paraständig mit je einer Gruppe K¹, K² oder K³ und L¹, L², oder L³ verknüpft und tragen keine weiteren Substituenten.

Weiterhin bevorzugt sind Verbindungen der allgemeinen Formel (I)
worin
- L¹, L² und L³: unabhangig voneinander 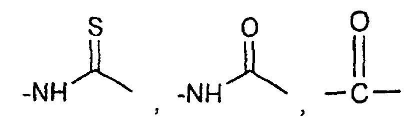 oder 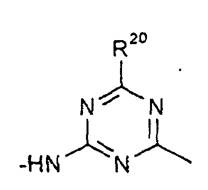 bedeuten, wobei
- R²⁰: für Chlor oder für Hydroxyalkylamino mit bis zu 6 Kohlenstoffatomen steht.

Besonders bevorzugt stehen L¹, L² und L³ für

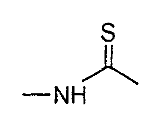

Weiterhin bevorzugt sind Verbindungen der allgemeinen Formel (I), worin M für ein Peptid steht, das über eine Aminofunktion mit L¹, L² und/oder L³ verknüpft ist, über eine Acylfunktion mit Cp verknüpft ist und dessen Aminosäurebausteine gegebenenfalls Schutzgruppen tragen können. Besonders bevorzugt sind Mono-, Di- und Tripeptide, insbesondere Mono- und Dipeptide.

Die Aminosäurebausteine werden bevorzugt ausgewählt aus der Gruppe Glycyl, Alanyl, Valyl, Leucyl, Lysyl, Seryl, Glutamyl, Threonyl, Asparagyl, Isoleucyl, Diaminopropionyl, Diaminobutyryl, Histidyl, Arginyl und/oder Omithyl.

Besonders bevorzugt sind die Aminosäurebausteine Glycyl, Alanyl, Valyl, Leucyl, Lysyl, Seryl, Asparagyl, Histidyl und/oder Glutamyl.

Die erfindungsgemäßen Verbindungen können in stereoisomeren Formen, beispielsweise als Enantiomere oder Diastereomere, oder als deren Gemische, beispielsweise als Racemat, vorliegen Die Erfindung betrifft sowohl die reinen Stereoisomere als auch deren Gemische.

Stereoisomerengemische können, falls erforderlich, in bekannter Weise in die stereoisomer einheitlichen Bestandteile getrennt werden, beispielsweise durch Chromatographie oder durch Kristallisationsverfahren.

Die erfindungsgemäßen Verbindungen können bedingt durch eine Rotationshinderung in rotationsisomeren Formen oder als deren Gemisch auftreten. Die Erfindung betrifft sowohl die reinen Rotationsisomere als auch deren Gemische

Rotationsisomerengemische können gegebenenfalls, falls erforderlich, mittels bekannter Methoden in die einheitlichen Bestandteile getrennt werden, beispielsweise durch Chromatographie (z B HPLC) oder durch Kristallisationsverfahren.

Möglich ist dies nicht nur auf der Endstufe der Glycokonjugate, sondern gegebenenfalls auch auf Zwischenstufen. Aus den rotamerenreinen Zwischenstufen können gegebenenfalls durch geeignete Syntheseführung die rotamerenreinen Endstufen hergestellt werden.

Die Stereochemie am anomeren Zentrum der Kohlenhydratbausteine K¹, K² und/oder K³ kann α oder β sein. Durch die Stereochemie an den anderen Zentren kann sich die gluco-, manno-, galacto-, gulo-, rhamno- oder fuco-Konfiguration ergeben.

Die Aminosäurebausteine können ebenso wie die Kohlenhydratbausteine jeweils in der D- oder in der L-Form vorliegen.

Der Camptothecin-Baustein Cp kann in der 20(R)- oder in der 20(S)-Konfiguration oder als Gemisch dieser beiden steroisomeren Formen vorliegen. Bevorzugt ist die 20(S)-Konfiguration.

Bevorzugte Beispiele für den Camptothecin-Baustein sind:

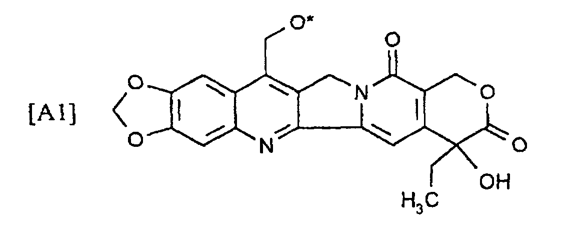

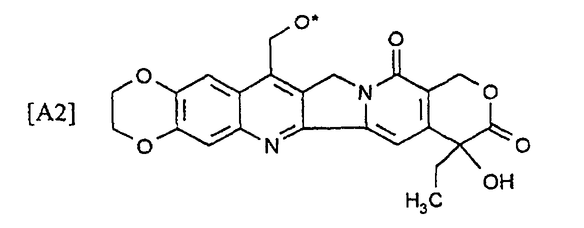

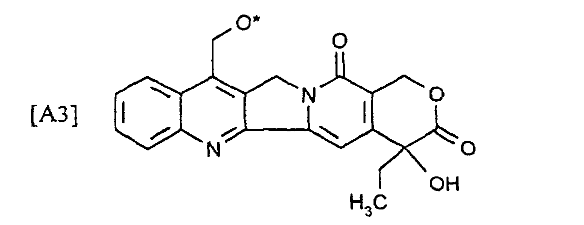

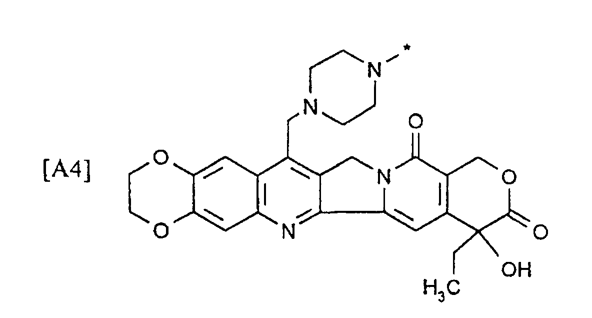

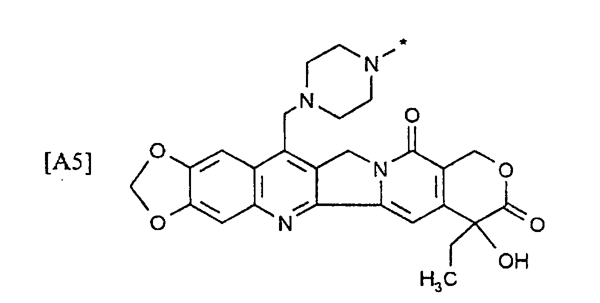

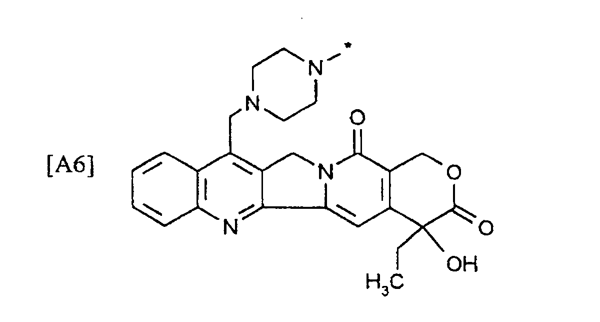

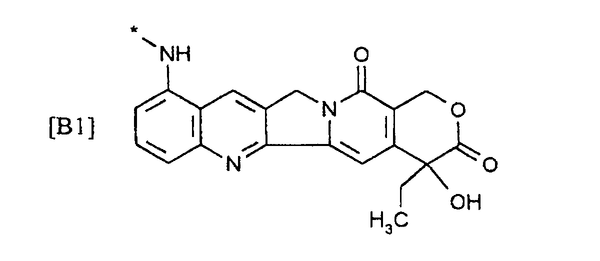

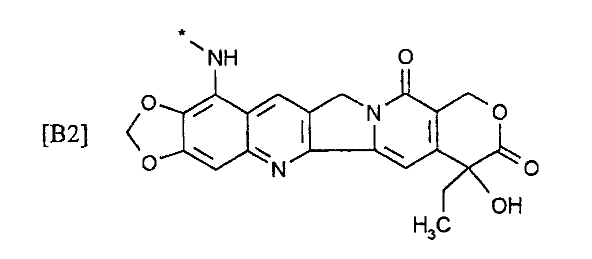

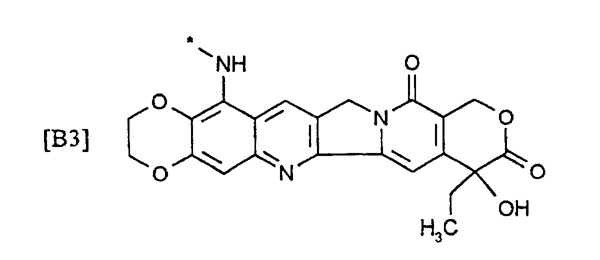

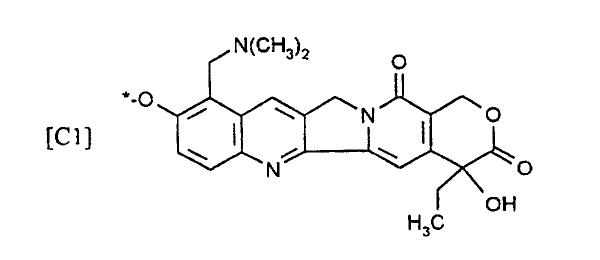

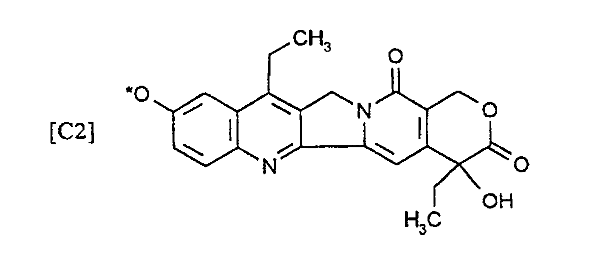

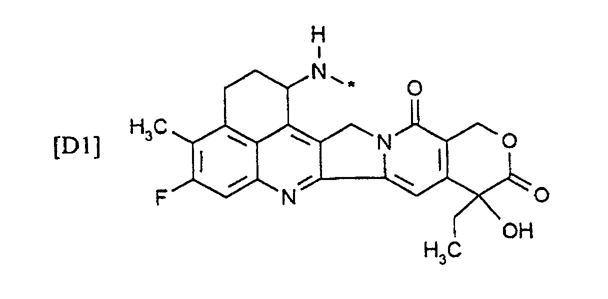

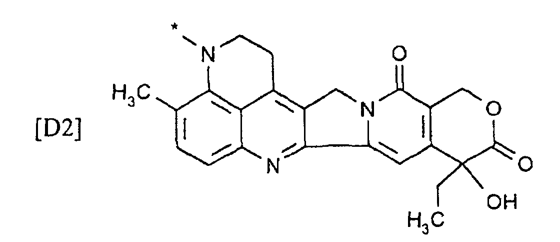

Von diesen beispielhaft genannten Camptothecin-Bausteinen besonders bevorzugt sind: [A1], [A3], [A4], [B1], [B2], [D1].

Durch Kombination der für die einzelnen Reste angegebenen bevorzugten oder besonders bevorzugten Bedeutungen ergeben sich entsprechende ganz besonders bevorzugte Verbindungen der allgemeinen Formel (I).

Die erfindungsgemäßen Verbindungen können auch in Form ihrer Salze vorliegen. Im allgemeinen seien hier Salze mit organischen oder anorganischen Basen oder Säuren sowie innere Salze genannt.

Zu den Säuren, die addiert werden konnen, gehören vorzugsweise Halogenwasserstoffsäuren, wie z.B. die Chlorwasserstoffsäure und die Bromwasserstoffsäure, insbesondere die Chlorwasserstoffsaure, ferner Phosphorsäure, Salpetersäure, Schwefelsäure, mono- und bifunktionelle Carbonsäuren und Hydroxycarbonsäuren, wie z.B. Essigsäure, Trifluoressigsäure, Maleinsäure, Malonsäure, Oxalsäure, Gluconsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak oder organischen Aminen wie beispielsweise Ethylamin, Di- bzw. Triethylamin, Di- bzw. Triethanolamin, Dicyclohexylamin, Dimethylaminoethanol, Arginin, Lysin, Ethylendiamin oder Phenethylamin.

Die erfindungsgemäßen Glycokonjugate können beispielsweise hergestellt werden durch Verknüpfung von amino- oder hydroxymodifizierten Camptothecin-Derivaten mit aktivierten Carboxylkomponenten, die ihrerseits beispielsweise Teile von geschützten Aminosäuren, Peptiden oder kohlenhydratmodifizierten Peptiden darstellen können.

Die Erfindung betrifft daher weiterhin ein Verfahren zur Herstellung von Verbindungen der allgemeinen Formel (I), dadurch gekennzeichnet, daß man Verbindungen der allgemeinen Formel (III)

Cp-H (III)

worin Cp die oben angegebene Bedeutung hat und das Wasserstoffatom an den mit * bezeichneten Positionen sitzt, mit einer dem oben definierten Rest M entsprechenden aktivierten Carboxylkomponente Ma, die gegebenenfalls Schutzgruppen trägt, in einem geeigneten Lösungsmittel, gegebenenfalls in Gegenwart einer Base, nach üblichen Methoden umsetzt, gegebenenfalls mittels bekannter Methoden selektiv eine, mehrere oder alle Schutzgruppen von M abspaltet und das Produkt mit Verbindungen der allgemeinen Formel (IV)

K¹-Sp¹-L¹a (IV)

worin K¹ und Sp¹ wie oben definiert sind und L¹a für eine reaktive Vorstufe der Gruppe L¹ steht, umsetzt, wobei gegebenenfalls die Schutzgruppen selektiv abgespalten und schrittweise verschiedene Gruppen K²-Sp²-L²- und K³-Sp³-L³- in vergleichbarer Weise eingeführt werden können
oder daß man, falls M ein Peptid bedeutet, in vergleichbarer Weise nach üblichen Methoden einen ersten Aminosäurerest in Form der entsprechenden gegebenenfalls Schutzgruppen tragenden aktivierten Carboxylkomponente einführt, gegebenenfalls Schutzgruppen abspaltet, weiterhin gegebenenfalls Schutzgruppen tragende Aminosäurereste anknüpft, gegebenenfalls erneut Schutzgruppen abspaltet, wie oben angegeben Reste der Formeln K¹-Sp¹-L¹, K²-Sp²-L² und/oder K³-Sp³-L³-einführt und falls erforderlich Schutzgruppen abspaltet.

Die Reaktionen können bei verschiedenen Druck- und Temperaturverhältnissen, beispielsweise 0,5 bis 2 bar, bzw. -30 bis +100°C, in geeigneten Lösungsmitteln wie Dimethylformamid (DMF), Tetrahydrofuran (THF), Dichlormethan, Chloroform, niederen Alkoholen, Acetonitril, Dioxan, Wasser oder in Gemischen der genannten Lösungsmittel durchgeführt werden. In der Regel sind Reaktionen in DMF oder THF/ Dichlormethan bei Normaldruck und bei einer Temperatur von 0 bis 60°C, insbesondere bei etwa Raumtemperatur, bevorzugt.

Fur die Aktivierung der Carboxylgruppen kommen die in der Peptidchemie bekannten Kupplungsreagenzien wie sie z.B. in Jakubke/Jeschkeit: Aminosäuren, Peptide, Proteine; Verlag Chemie 1982 oder Tetrahedr. Lett. 34, 6705 (1993) beschrieben sind, in Frage. Bevorzugt sind beispielsweise Säurechloride, N-Carbonsäureanhydride oder gemischte Anhydride.

Weiterhin geeignet zur Aktivierung der Carboxylgruppen ist die Bildung von Addukten mit Carbodiimiden z.B. N,N'-Diethyl-, N,N'-Diisopropyl-, N,N'-Dicyclohexylcarbodiimid, N-(3-Dimethylaminopropyl)-N'-ethyl-carbodiimid-Hydrochlorid, N-Cyclohexyl-N'-(2-morpholinoethyl)-carbodiimid-metho-p-toluolsulfonat, oder Carbonylverbindungen wie Carbonyldiimidazol, oder 1,2-Oxazoliumverbindungen wie 2-Ethyl-5-phenyl-1,2-oxazolium-3-sulfat oder 2-tert-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroform, oder Benzotriazolyloxy-tris(dimethylamino)phosphonium-hexafluorophosphat, 1-Hydroxybenzotriazol oder N-Hydroxysuccinimid.

Als Basen können beispielsweise Triethylamin, Ethyl-diisopropylamin, Pyridin, N,N-Dimethylaminopyridin oder andere eingesetzt werden.

Als Schutzgruppen für eventuelle Drittfu; tioner der Aminosäuren oder Nicht-Verknüpfungspositionen im Camptothecin-Teil können die in der Peptidchemie bekannten Schutzgruppen beispielsweise vom Urethan-, Alkyl-, Acyl-, Ester- oder Amid-Typ eingesetzt werden.

Aminoschutzgruppen im Rahmen der Erfindung sind die üblichen in der Peptid-Chemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, tert.-Butoxycarbonyl (Boc), Allyloxycarbonyl, Vinyloxycarbonyl, 3,4,5-Trimethoxybenzyloxycarbonyl, 2,2,2-Trichlorethoxycarbonyl, 2,2,2-Trichlor-tert-butoxycarbonyl, Menthyloxycarbonyl, 4-Nitrophenoxycarbonyl, Fluorenyl-9-methoxycarbonyl (Fmoc), Formyl, Acetyl, Propionyl, Pivaloyl, 2-Chloracetyl, 2-Bromacetyl, 2,2,2-Trifluoracetyl, 2,2,2-Trichloracetyl, Benzoyl, Benzyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl, 4-Nitrophenyl oder 2-Nitrophenylsulfenyl. Besonders bevorzugt sind die Fmoc-Gruppe und die Boc-Gruppe.

Bevorzugte Carboxylschutzgruppen sind lineare oder verzweigte C₁-C₄-Alkylester.

Die Modifizierung der mit einer Brücken-Gruppierung M verknüpften Camptothecin-Derivate mit Kohlenhydratresten kann über verschiedene Methoden und Linkergruppen erfolgen. Bevorzugt ist z.B. die Überführung von p-Aminophenylglycosiden in Isothiocyanate und die Verknüpfung beispielsweise mit Aminogruppen. Weiterhin können auch Carboxyalkyl- oder Aminoalkylglycoside leicht mit Amino- bzw. Carboxylgruppen gekuppelt werden.

Die Abspaltung von Schutzgruppen in entsprechenden Reaktionsschritten kann zum Beispiel durch Säure- oder Base-Einwirkung, hydrogenolytisch oder auf andere Weise reduktiv erfolgen.

### Biologische Testung

### 1. Wachstumsinhibitionstest zur Bestimmung der cytotoxischen Eigenschaften:

Die humanen Dickdarmzellinien SW 480 und HT 29 (ATCC-Nr. CCL 228 und HBT-38) sowie die Maus-Melanom-Zellinie B 16 F 10 wurden in Rouxschalen in RPMI 1640 Medium unter Zusatz von 10 % FCS gezogen. Anschließend wurde trypsiniert und in RPMI plus 10 % FCS zu einer Zellzahl von 50.000 Zellen/ml aufgenommen. 100 µl Zellsuspension/ Well wurden in eine 96 Mikrowellplatte gegeben und 1 Tag bei 37°C im CO₂ Brutschrank inkubiert. Anschließend wurden weitere 100 µl RPMI Medium und 1 µl DMSO mit den Prüfsubstanzen zugesetzt Das Wachstum wurde nach Tag 3 und Tag 6 überprüft. Dazu wurde zu jedem Mikrowell 40 µl MTT-Lösung (3-(4,5-Dimethylthiazol-2-yl)-2,5-diphenyltetrazolinbromid) mit einer Ausgangskonzentration von 5 mg/ml H₂O zugesetzt. Es wurde 5 Stunden im CO₂-Brutschrank bei 37°C inkubiert. Anschließend wurde das Medium abgesaugt und 100 µl i-Propanol/Well zugesetzt. Nach 30 min Schütteln mit 100 µl H₂O wurde die Extinktion bei 540 nm mit einem Titertek Multiskan MCC/340 (Flow) gemessen.

Die cytotoxische Wirkung ist in der Tabelle 1 als IC₅₀-Wert (Dosis, welche das Zellwachstum auf 50 % des Zellwachstums der Kontrolle inhibiert) jeweils für die SW 480- und HT 29- und B16F10-Zellinie angegeben:

**Tabelle 1:**

| Beispiel | IC₅₀ / µM SW 480 | IC₅₀ / µM HT 29 | IC₅₀ / µM B16F10 |
|---|---|---|---|
| 1.1 | 0,015 | 0,025 | 0,18 |
| 1.3 | 0,04 | 0,03 | n.d. |
| 1.4 | 0,03 | 0,04 | 0,2 |
| 2.1 | 0,2 | 0,06 | 2 |
| 3.1 | 0,08 | 0,06 | 0,5 |
| 3.3 | 0,25 | 0,1 | n.d. |
| 3.5 | 0,3 | 0,3 | n.d. |
| 4.1 | 0,03 | 0,04 | n.d. |

### 2. Hämatopoetische Aktivität von Glycokonjugaten im Vergleich zum zugrundeliegenden Wirkstoff:

### Material und Methoden

Knochenmarkzellen werden aus dem Femur der Maus gespült. 10⁵-Zellen werden in McCoy 5A-Medium (0,3 % Agar) zusammen mit rekombinantem murinem GM-CSF (Genzyme; Stammzellen-Kolonienbildung) und den Substanzen (10⁻⁴ bis 100 µg/ml) bei 37°C und 7 % CO₂ inkubiert. 7 Tage später werden die Kolonien (<50 Zellen) und Kluster (17-50 Zellen) ausgezählt.

### Ergebnisse:

Wie in Tab. 2 am Beispiel 2.1 dargestellt, zeigen die untersuchten Glycokonjugate gegenüber dem zugrundeliegenden Wirkstoff eine etwa um den Faktor 100 verminderte Hemmung der Knochenmarkstammzellproliferation.

**Tabelle 2:**

| Beispiel | IC₅₀ [µg/ml] |
|---|---|
| 2.1.a | 0,0016 |
| 2.1 | 0,17 |

### 3. In-vivo-Hemmung des Tumorwachstums im Nacktmaus-Modell

### Material:

Für alle in-vivo-Experimente zur Untersuchung der Hemmung des Tumorwachstums wurden athymische Nacktmäuse (NMRI nu/nu-Stamm) verwendet. Das ausgewählte großzellige Lungenkarzinom LXFL 529 wurde durch serielle Passage in Nacktmäusen entwickelt. Der menschliche Ursprung des Tumors wurde durch isoenzymatische und immunohistochemische Methoden belegt.

### Experimenteller Aufbau:

Der Tumor wurde subcutan in beide Flanken von 6 bis 8 Wochen alten nu/nu-Nacktmäusen implantiert. Die Behandlung wurde, abhängig von der Verdopplungszeit, gestartet sobald die Tumoren einen Durchmesser von 5 - 7 mm erreicht hatten. Die Mäuse wurden der Behandlungsgruppe und der Kontrollgruppe (5 Mäuse pro Gruppe mit 8 - 10 auswertbaren Tumoren) durch Randomisieren zugeteilt. Die einzelnen Tumoren der Kontrollgruppe wuchsen alle progressiv.

Die Größe der Tumoren wurde in zwei Dimensionen mittels Schieblehre vermessen. Das Tumorvolumen, das gut mit der Zellzahl korreliert, wurde anschließend für alle Auswertungen verwendet. Das Volumen wurde gemäß der Formel "Länge x Breite x Breite / 2" berechnet ([a x b²] / 2, a bzw. b stehen für zwei rechtwinklig angeordnete Durchmesser).

Die Werte des relativen Tumorvolumens (RTV) wurden für jeden einzelnen Tumor durch Dividieren der Tumorgröße am Tag X mit der Tumorgröße des Tages 0 (zum Zeitpunkt der Randomisierung) berechnet. Die mittleren Werte des RTV wurden dann für die weitere Auswertung verwendet.

Die Hemmung der Zunahme des Tumorvolumens (Tumorvolumen der Testgruppe/ Kontrollgruppe, T/C, in Prozent) war der abschließende Meßwert.

### Behandlung:

Die Applikation der Verbindungen erfolgte an Tag 1, 2 und 3 nach Randomisierung intraperitoneal (i.p).

### Ergebnisse:

Anhand der Verbindung aus Beispiel 1.1 ist die therapeutische Wirksamkeit der erfindungsgemäßen Glycokonjugate gegenüber dem großzelligen humanen Lungentumorxenografts LXFL 529 dargestellt. Die Therapie führt bei der maximal tolerablen Dosis (MTD), bei 1/2 MTD und bei 1/4 MTD zu einer Komplettremission des Tumors.

**Tabelle 3:**

| Therapie | Dosis [mg/kg/Tag] | Überlebenszeit [Tage] | | Zahl der Tumoren | relatives Tumorvolumen an Tag 14 [% des Tages 0] | relatives Körpergewicht an Tag 7 [%des Tages 0] |
|---|---|---|---|---|---|---|
| Kontrollgruppe | - | >21 >21 >21 | >21 >21 | 10 | 691 | 102 |
| 1.1 | 12,5 (MTD) | >21 >21 >21 | >21 >21 | 9 | 0,1 | 72 |
| 1.1 | 6,25 | >21 >21 >21 | >21 >21 | 10 | 0,1 | 93 |
| 1.1 | 3,125 | >21 >21 >21 | >21 >21 | 9 | 0,1 | 93 |

Die erfindungsgemäßen Verbindungen weisen sowohl in-vitro als auch in-vivo eine überraschend starke Antitumor-Wirksamkeit gegenüber verschiedenen Tumoren, insbesondere solchen der Lunge und des Dickdarms, verbunden mit einer großen Selektivität gegenüber nicht malignen Zellen auf.

Sie eignen sich daher zur Behandlung von Krebserkrankungen, insbesondere von solchen der Lunge und des Dickdarms.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Der oder die Wirkstoffe können gegebenenfalls in einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30mg/kg Körpergewicht.

### Beispiele:

### Kohlenhydrat-Edukte

### Beispiel 1.1:

### p-Aminophenyl-3-O-methyl-β-L-fucopyranosid

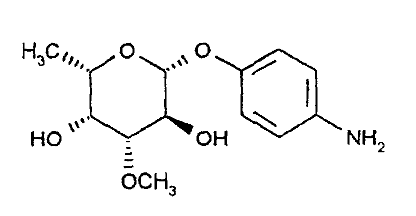

### I.1.a) p-Nitrophenyl-3-O-methyl-β-L-fucopyranosid:

6 g (21mmol) p-Nitrophenyl-β-L-fucopyranosid in 300ml absol. Methanol werden mit 7,84g (31,5mmol) Dibutylzinnoxid versetzt und 2h unter Rückfluß erhitzt. Anschließend wird eingeengt, der Rückstand getrocknet und dann in 300ml DMF aufgenommen. Nach Zugabe von 15,7ml Methyliodid wird der Ansatz 40h bei 70°C gerührt. Das Lösungsmittel wird im Vakuum entfernt und der Rückstand in 300ml Dichlormethan aufgenommen. Die Suspension wird filtriert, die verbleibende Lösung erneut eingeengt und einer Flash-Chromatographie (Dichlormethan/Methanol 99:1) unterzogen. Nach Einengen erhält man 3,82g (61%) des Zielproduktes.

### I.1) p-Aminophenyl-3-O-methyl-β-L-fucopyranosid:

3,81g (12,73mmol) p-Nitrophenyl-3-O-methyl-β-L-fucopyranosid werden in Methanol gelöst und nach Zusatz von Platindioxid in einer Wasserstoffatmosphäre bei geringem Überdruck hydriert. Nach Abfiltrieren des Katalysators und Fällen mit Ether erhält man 3g (88%) des Zielprodukts. [DC: Dichlormethan/Methanol 9:1 R_{f} = 0,53].

### Beispiel I.2:

### p-Aminophenyl-3-O-carboxymethyl-β-L-fucopyranosid

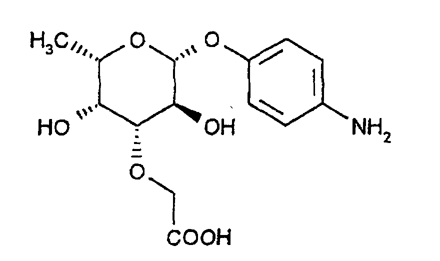

### I.2.a) p-Nitrophenyl-3-O-methoxycarbonylmethyl-β-L-fucopyranosid:

1g (3,5mmol) p-Nitrophenyl-β-L-fucopyranosid und 1,3g (5,2mmol) Dibutylzinnoxid werden in 50ml Methanol 2h unter Rückfluß erhitzt. Die Lösung wird eingeengt, der Rückstand in 50ml Dioxan aufgenommmen, mit 2ml Bromessigsäure-methylester sowie 100mg Tetrabutylammoniumiodid versetzt und 16h unter Rückfluß erhitzt. Das Lösungsmittel wird abgedampft und das Produkt durch Flash-Chromatographie (Dichlormethan/Methanol 99:1) gereinigt. Nach Einengen der entsprechenden Fraktionen und Fällen aus Methanol/Ether erhält man 455mg (37%) der Zielverbindung.

### I.2) p-Aminophenyl-3-O-carboxymethyl-β-L-fucopyranosid:

282mg (0,79mmol) p-Nitrophenyl-3-methoxycarbonylmethyl-β-L-fucopyranosid werden in 20ml Methanol gelöst und mit 440µl einer wäßrigen 2N Lithiumhydroxid-Lösung versetzt. Nach 2h Rühren bei Raumtemp. wird mit saurem Ionenaustauscher SC108 auf pH 3 eingestellt und filtriert. Zum Filtrat werden 250mg Palladium auf Aktivkohle zugesetzt. Anschließend wird 1,5h mit Wasserstoff bei geringem Überdruck hydriert, der Katalysator abgetrennt und mit Methanol gewaschen. Einengen, Aufnehmen in Wasser und Gefriertrocknung führen in 86%iger Ausbeute zum Zielprodukt (212mg). [DC: Acetonitril/Wasser/Eisessig 5:1:0,2 R_{f}= 0,24]

Als weitere Kohlenhydratbausteine können beispielsweise die folgenden Derivate eingesetzt werden:
p-Aminophenyl-β-L-fucopyranosid
p-Aminophenyl-2-O-methyl-β-L-fucopyranosid
p-Aminophenyl-2-O-hydroxyethyl-β-L-fucopyranosid
p-Aminophenyl-4-O-methyl-β-L-fucopyranosid
p-Aminophenyl-3-O-methyl-α-L-fucopyranosid
p-Aminophenyl-3-O-n-propyl-β-L-fucopyranosid
p-Aminophenyl-3-desoxy-β-L-fucopyranosid
p-Aminophenyl-3,4-didesoxy-β-L-fucopyranosid
p-Aminophenyl-3,4-epoxy-β-L-fucopyranosid
p-Aminophenyl-4-desoxy-β-L-fucopyranosid
p-Aminophenyl-3-O-methoxycarbonylmethyl-β-L-fucopyranosid
p-Aminophenyl-3-O-hydroxyethyl-β-L-fucopyranosid
p-Aminophenyl-2-O-carboxymethyl-β-L-fucopyranosid
p-Aminophenyl-3-O-succinyl-β-L-fucopyranosid
p-Aminophenyl-3,4-di-O-methyl-β-L-fucopyranosid
p-Aminophenyl-3-O-carbamoylmethyl-β-L-fucopyranosid
p-Aminophenyl-α-L-rhamnopyranosid
p-Aminophenyl-β-D-galactopyranosid
p-Aminophenyl-2-O-methyl-β-D-galactopyranosid
p-Aminophenyl-3-O-methyl-β-D-galactopyranosid
p-Aminophenyl-4-O-methyl-β-D-galactopyranosid
p-Aminophenyl-6-O-methyl-β-D-galactopyranosid
p-Aminophenyl-2,3-di-O-methyl-β-D-galactopyranosid
p-Aminophenyl-2,4-di-O-methyl-β-D-galactopyranosid
p-Aminophenyl-2,6-di-O-methyl-β-D-galactopyranosid
p-Aminophenyl-3,4-di-O-methyl-β-D-galactopyranosid, Acetat
p-Aminophenyl-3,6-di-O-methyl-β-D-galactopyranosid
p-Aminophenyl-4,6-di-O-methyl-β-D-galactopyranosid
p-Aminophenyl-2,3,4-tri-O-methyl-β-D-galactopyranosid
p-Aminophenyl-2,3,6-tri-O-methyl-β-D-galactopyranosid
p-Aminophenyl-2,4,6-tri-O-methyl-β-D-galactopyranosid
p-Aminophenyl-3,4,6-tri-O-methyl-β-D-galactopyranosid
p-Aminophenyl-3-desoxy-β-D-galactopyranosid
p-Aminophenyl-3,4-didesoxy-β-D-galactopyranosid
p-Aminophenyl-6-O-acetyl-β-D-galactopyranosid
p-Aminophenyl-3-O-methoxycarbonylmethyl-β-D-galactopyranosid
p-Aminophenyl-3-O-carboxymethyl-β-D-galactopyranosid, Natriumsalz
p-Aminophenyl-3-O-carbamoylmethyl-β-D-galactopyranosid
p-Aminophenyl-3-O-(N-methyl-carbamoylmethyl)-β-D-galactopyranosid
p-Aminophenyl-α-D-mannopyranosid
p-Aminophenyl-3-O-methyl-α-D-mannopyranosid
p-Aminophenyl-2,3-di-O-methyl-α-D-mannopyranosid
p-Aminophenyl-3-O-methoxycarbonylmethyl-α-D-mannopyranosid
p-Aminophenyl-3-O-carboxymethyl-α-D-mannopyranosid
p-Aminophenyl-3-O-carbamoylmethyl-α-D-mannopyranosid
p-Aminophenyl-4-O-(b-D-galactopyranosyl)-β-D-glucopyranosid
p-Aminophenyl-4-O-(3'-sulfat-β-D-galactopyranosyl)-β-D-glucopyranosid, Natriumsalz
p-Aminophenyl-4-O-(3'-O-methyl-β-D-galactopyranosyl)-β-D-glucopyranosid
p-Aminophenyl-2-O-methyl-4-O-(3'-O-methyl-β-D-galactopyranosyl)-β-D-glucopyranosid
p-Aminophenyl-4-O-(3',4'-di-O-methyl-β-D-galactopyranosyl)-β-D-glucopyranosid

Bereits in EP 501 250 wurde die Synthese der folgenden Kohlenhydratbausteine beschrieben:
Carboxymethyl-β-L-fucopyranosid
5-Carboxypentyl-β-L-fucopyranosid

Die Verknüpfung dieser beiden Bausteine mit Peptidkonjugaten kann in der in EP 501 250 beschriebenen Weise erfolgen.

### Glycokonjugate

### Beispiel 1.1:

### 7-{N^{α},N^{ε}-bis-[O-(3-O-Methyl-β-L-fucopyranosyl)-4-hydroxy-phenylaminothiocarbonyl]-L-lysyl-L-alanyloxymethyl}-camptothecin

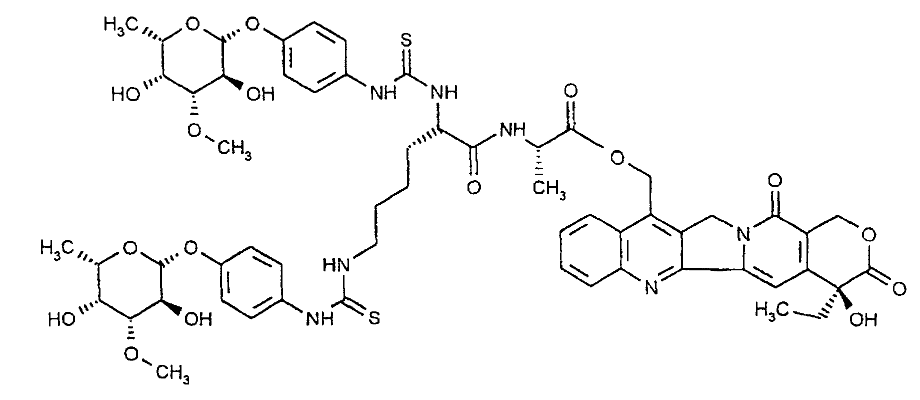

### 1.1.a) 7-Hydroxymethyl-camptothecin:

Diese Verbindung wird nach der Vorschrift von Miyasaka et al. (Chem. Pharm. Bull. 39 (1991) 2574) hergestellt.

### 1.1.b) 7-(L-Alanyloxymethyl)-camptothecin, Trifluoracetat:

1g (2,64mmol) 7-Hydroxymethyl-camptothecin werden in 100ml DMF gelöst und dann mit 100mg 4-N,N-Dimethylaminopyridin und 1,5 Äquivalenten N-tert-Butoxycarbonyl-L-alanin-N-carboxy-anhydrid versetzt und die Suspension 16h bei Raumtemperatur gerührt. Man engt ein und reinigt durch Flash-Chromatographie an Ethylacetat/Petrolether 1:1 und später 1,5:1. Das gereinigte Material wird in 30ml Dichlormethan aufgenommen und bei 0°C mit 5ml Trifluoressigsäure versetzt. Nach 30min Rühren wird eingeengt und das aminodeblockierte Produkt aus Dichlormethan/Ether gefällt. Anschließend wird aus Dioxan/Wasser lyophilisiert. Man erhält die Zielverbindung in einer Gesamtausbeute von 59%. [FAB-MS: m/z = 450 = M+H].

### 1.1.c) 7-(L-Lysyl-L-alanyloxymethyl)-camptothecin, Bis-Trifluoracetat:

200mg des Konjugats aus Beispiel 1.1.b werden zu einer Lösung aus 150mg (1,2Äq.) N^{α},N^{ε}-bis-(tert-Butoxycarbonyl)-L-lysin, 88mg N-Hydroxybenzotriazol und 100mg N-Ethyl-N'-(3-dimethylaminopropyl)-carbodiimid Hydrochlorid in 20ml Dimethylformamid gegeben und eine Stunde bei Raumtemperatur gerührt. Man engt ein, nimmt in Dichlormethan auf und extrahiert dreimal mit Wasser Nach Trocknen der organischen Phase wird eingeengt und aus Methanol/Ether gefällt. Anschließend wird das erhaltene Produkt in 15ml Dichlormethan aufgenommen, bei 0°C mit 1ml Trifluoressigsäure versetzt und eine Stunde bei Raumtemp. gerührt. Dann setzt man erneut 1ml Trifluoressigsäure zu und rührt eine weitere Stunde. Nach Einengen und Fällen aus Dichlormethan/Ether erhält man die Zielverbindung in 65%iger Ausbeute. [DC: Acetonitril/Wasser/Eisessig 10:5:3 R_{f} = 0,44]

### 1.1) 7-{N^{α},N^{ε}-bis-[O-(3-O-Methyl-β-L-fucopyranosyl)-4-hydroxyphenylamino-thiocarbonyl]-L-lysyl-L-alanyloxymethyl}-camptothecin

Eine Lösung von Verbindung I.1 (0,35mmol) in Dioxan/Wasser 1:1 (15ml) wird unter Rühren mit Thiophosgen (0,5mmol) versetzt. Nach 10 min. versetzt man mit 4 Äquivalenten Ethyldiisopropylamin, engt anschließend im Vakuum ein und trocknet den Rückstand für 1h im Ölpumpenvakuum. Das erhaltene Isothiocyanat wird in absolutem Dimethylformamid (10ml) gelöst und mit Verbindung 1.1.c (0,15mmol) sowie mit 4 Äquivalenten Ethyldiisopropylamin versetzt. Man rührt für 16h bei Raumtemperatur, engt dann im Vakuum ein und verrührt den Rückstand mit 20ml Wasser. Man saugt ab, nimmt den Rückstand in Dichlormethan/Methanol auf und fällt mit Ether. Nach erneuter Fällung erhält man das Zielprodukt in einer Ausbeute von 61%. [DC: Acetonitril/Wasser 10:1 R_{f}= 0,45] [FAB-MS: m/z = 1200 = M+H].

### Beispiel 1.2

### 7-{N^{α}-[O-(3-O-Carboxymethyl-β-L-fucopyranosyl)-4-hydroxy-phenylaminothiocarbonyl]-L-lysyl-L-alanyloxymethyl}-camptothecin, Hydrochlorid

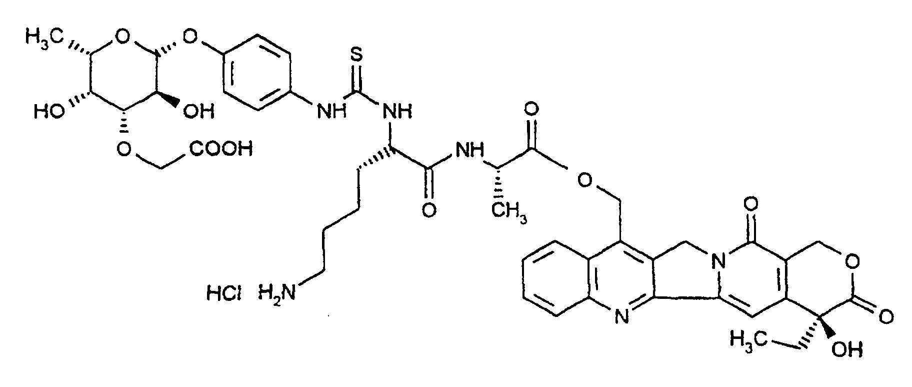

### 1.2.a) 7-[N^{ε}-(Fluorenyl-9-methoxycarbonyl)-L-lysyl-L-alanyloxymethyl]-camptothecin, Trifluoracetat:

Das Konjugat aus Beispiel 1.1.b wird nach Standard-Vorschrift mit N^{α}-(tert-Butoxycarbonyl)-N^{ε}-(fluorenyl-9-methoxycarbonyl)-L-lysin verknüpft und anschließend an der α-Aminofunktion deblockiert

### 1.2.b) 7-{N^{α}-[O-(3-O-Carboxymethyl-β-L-fucopyranosyl)-4-hydroxyphenylamino-thiocarbonyl]-N^{ε}-[fluorenyl-9-methoxycarbonyl]-L-lysyl-L-alanyloxymethyl}-camptothecin:

Die Verbindung aus Beispiel 1.2.a wird in Analogie zu Beispiel 1.1 mit 1,2 Äquivalenten des Kohlenhydratderivats aus Beispiel I.2 umgesetzt.

### 1.2) 7-{N^{α}-[O-(3-O-Carboxymethyl-β-L-fucopyranosyl)-4-hydroxyphenylamino-thiocarbonyl]-L-lysyl-L-alanyloxymethyl}-camptothecin, Hydrochlorid:

Das Konjugat 1.2.b wird mit Piperidin in DMF deblockiert. Nach 30min, engt man ein und digeriert den Rückstand zweimal mit Dichlormethan. Dann wird in DMF aufgenommen und mit Methanol/Ether gefällt. Das Produkt wird abgesaugt, mit Ether gewaschen und dann aus Dioxan/Wasser lyophilisiert. Anschließend nimmt man in Wasser auf, versetzt mit 1 Äquivalent einer 0,01N HCI-Lösung und lyophilisiert erneut.

### Beispiel 1.3:

### 7-{N^{α}-[O-(3-O-Carboxymethyl-β-L-fucopyranosyl)-4-hydroxy-phenylaminothiocarbonyl]-L-lysyl-L-valyloxymethyl)-camptothecin, Hydrochlorid

Die Verbindung wird analog zu Beispiel 1.2 hergestellt, nachdem 7-Hydroxymethyl-Camptothecin zuvor mit N-tert-Butoxycyrbonyl-L-valin-N-carboxyanhydrid analog zu Beispiel 1.1.b acyliert wurde. [DC: Acetonitril/Wasser/Eisessig 10:3:1,5 R_{f} = 0,3] [FAB-MS: m/z = 961 = M+H].

### Beispiel 1.4:

### 7-{N^{α}-[O-(3-O-Methyl-β-L-fucopyranosyl)-4-hydroxy-phenylaminothiocarbonyl)-L-lysyl-L-valyloxymethyl}-camptothecin, Hydrochlorid

Die Verbindung wird analog zu Beispiel 1.3 hergestellt. Als Kohlenhydratkomponente wird die Verbindung aus Beispiel 1.1 anstelle derer aus Beispiel 1.2 eingesetzt [DC: Acetonitril/Wasser/Eisessig 5:1:0,2 R_{f} = 0,34].

### Beispiel 2.1:

### 9-{N^{α},N^{ε}-bis-[O-(3-O-Methyl-β-L-fucopyranosyl)-4-hydroxy-phenylaminothiocarbonyl]-L-lysyl-L-alanyl-amino}-camptothecin

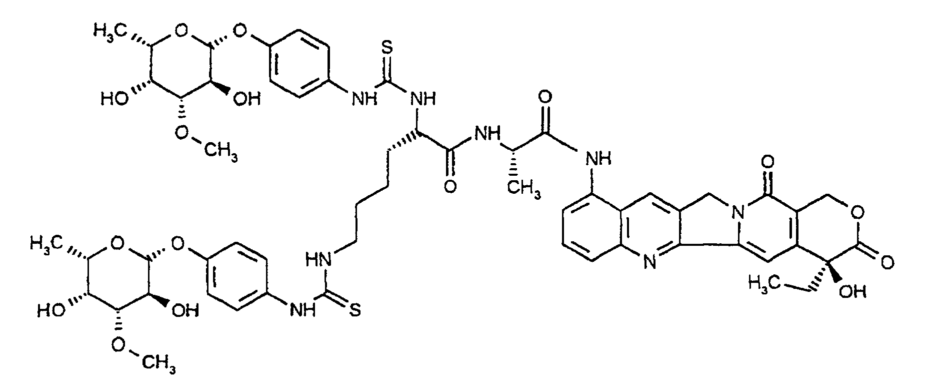

### 2.1.a) 9-Amino-camptothecin:

Es wird nach der Vorschrift von Wani et al. (J.Med.Chem. 29 (1986), 2358) hergestellt.

### 2.1.b) 9-[L-alanyl-amino}-camptothecin

N-(Fluorenyl-9-methoxycarbonyl)-L-alanin (0,28 mmol) werden in bekannter Weise mit Thionylchlorid in das Säurechlorid überführt. Dieses wird nach kurzem Trocknen unter Schutzgas zu einer Lösung aus 0,14mmol der Verbindung 2.1.a und 17µl Pyridin in 20ml abs. Dichlormethan gegeben. Nach 16h Rühren bei Raumtemp. engt man ein, nimmt den Rückstand in DMF auf und setzt 500mg Celite zu. Man dampft das Lösungsmittel ab und gibt das an Celite gebundene Material auf eine mit Kieselgel gefüllte Flash-Säule. Anschließend chromatographiert man mit 5% Methanol in Dichlormethan. Die entsprechenden Fraktionen werden gesammelt, eingeengt und mit Ether digeriert. Nach Trocknung erhält man die Fmoc-geschützte Zwischenstufe in 59 %iger Ausbeute. Das Produkt wird in 5ml DMF aufgenommen und zur Ablösung der Fmoc-Schutzgruppe mit 250µl Piperidin versetzt. Nach 20 min Rühren bei Raumtemp. engt man ein und fällt die Zielverbindung aus Dichlormethan mit Ether. Sie wird quantitativer Ausbeute erhalten. [DC: Acetonitril/Wasser/Eisessig 5:1:0,2 R_{f} = 0,2].

### 2.1.c) 9-(L-Lysyl-L-alanyl-amino)-camptothecin, Bis-Trifluoracetat:

100mg des Konjugats aus Beispiel 2.1.b werden in Analogie zu Beispiel I.1.c mit N^{α},N^{ε}-bis-(tert-Butoxycarbonyl)-L-lysin verknüpft und das Produkt anschließend mit Trifluoressigsäure deblockiert. Die Zielverbindung wird in 75%iger Gesamtausbeute erhalten. [DC: Acetonitril/Wasser/Eisessig 10:5:3 R_{f} = 0,19] [FAB-MS: m/z = 563 = M+H].

### 2.1) 9-{N^{α},N^{ε}-bis-[O-(3-O-Methyl-β-L-fucopyranosyl)-4-hydroxyphenylamino-thiocarbonyl]-L-lysyl-L-alanyl-amino}-camptothecin

In Analogie zu Beispiel 1.1 wird durch Kupplung des Peptidkonjugats 2.1.c mit dem Kohlenhydratderivat aus Beispiel I.1 das gewünschte Glycokonjugat hergestellt. Ausb.: 60% [DC: Acetonitril/Wasser 10:1 R_{f} = 0,33] [FAB-MS: m/z = 1185 = M+H]

### Beispiel 2.2

### 9-{N^{α}-[O-(3-O-Carboxymethyl-β-L-fucopyranosyl)-4-hydroxy-phenylaminothiocarbonyl]-L-lysyl-L-alanyl-amino}-camptothecin, Hydrochlorid

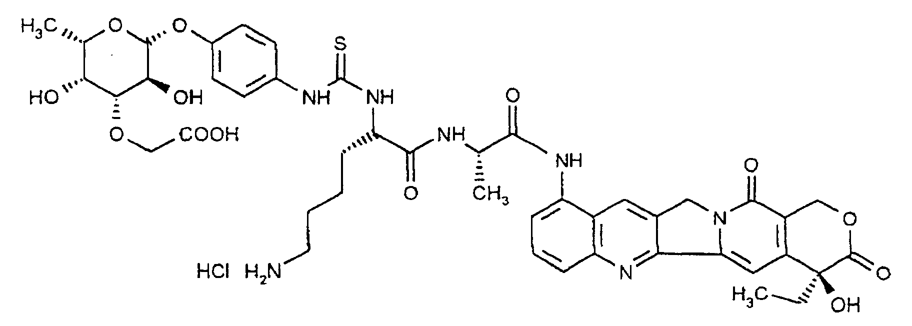

Dieses Konjugat wird in Analogie zu den Beispielen 1.2.a, 1.2.b und 1.2 aus dem L-Alanyl-Konjugat in Beispiel 2.1.b hergestellt.

### Beispiel 2.3:

### 9-{N^{α}-[O-(3-O-Carboxymethyl-β-L-fucopyranosyl)-4-hydroxy-phenylaminothiocarbonyl]-L-lysyl-L-valyl-amino}-camptothecin, Hydrochlorid

Die Verbindung wird analog zu Beispiel 2.2 hergestellt.

### Beispiel 3.1:

### 10,11-(Ethylendioxy)-7-{1-[(N^{α},N^{ε}-bis-(O-(3-O-methyl-β-L-fucopyranosyl)-4-hydroxy-phenylamino-thiocarbonyl)-L-lysyl-L-alanyl]-piperazino-4-yl-methyl}-20(R/S)-camptothecin

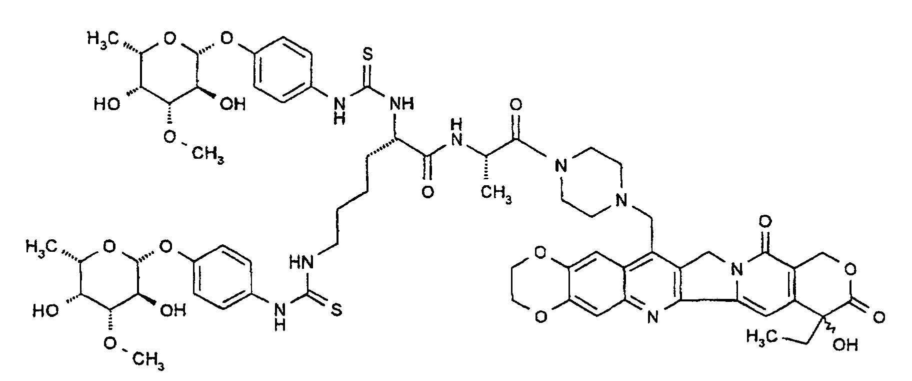

### 3.1.a) 7-(Chlormethyl)-10,11-(ethylendioxy)-20(R/S)-camptothecin:

Diese Verbindung wird in Analogie zu der 20(S)-Verbindung aus dem racemischen Tricyclus nach der Vorschrift von Luzzio et al. (J.Med.Chem. 38 (1995), 395) hergestellt.

### 3.1.b) N-(tert.-Butoxycarbonyl-L-alanyl)-piperazin

Man löst 1,15g des N-Hydroxysuccinimid-esters von N-(tert-Butoxycarbonyl)-L-alanin in 100ml DMF und tropft die Lösung über 6h in eine Mischung aus 3,4g (10 Equivalenten) Piperazin in 200ml DMF ein. Man engt ein, nimmt den Rückstand in Wasser auf und extrahiert dreimal mit je 200ml Dichlormethan. Die organische Phase wird getrocknet und das Lösungsmittel im Vakuum entfernt. Man erhält die Zielverbindung in einer Ausbeute von 88%. [DC: Acetonitril/Wasser/Eisessig 5:1:0,2 R_{f} = 0,42].

### 3.1.c) 10,11-(Ethylendioxy)-7-{1-[L-alanyl]-piperazin-4-yl-methyl}-20(R/S)-camptothecin, Trifluoracetat:

125mg (0,485mmol) der Verbindung 3.1.b werden in 2ml DMF aufgenommen und bei -50°C zu einer Lösung aus 100mg (0,22mmol) 3.1.a in 10ml DMF getropft. Man läßt auf Raumtemperatur erwärmen und 16h rühren. Anschließend reinigt man das entstandene Produkt durch Flash-Chromatographie an Kieselgel mit Dichlormethan/Methanol 98:2 und später 96:4. Nach Einengen der entsprechenden Fraktionen erhält man die geschutzte Zwischenstufe in einer Ausbeute von 44%. [DC: Dichlormethan/Methanol 95:5 R_{f} = 0,3]. Die Abspaltung der Boc-Schutzgruppe nach Standardbedingungen mit Trifluoressigsaure in Dichlormethan liefert die Zielverbindung in nahezu quantitativer Ausbeute. [DC: Acetonitril/Wasser/Eisessig 5:1:0,2 R_{f} = 0,16] [FAB-MS: m/z = 576 = M+H].

### 3.1.d) 10,11-(Ethylendioxy)-7-{1-[L-lysyl-L-alanyl]-piperazin-4-yl-methyl}-20(R/S)-camptothecin, Bis-Trifluoracetat:

50mg des Konjugats aus Beispiel 3.1.c werden zu einer Lösung aus 31mg (0,088mmol) N^{α},N^{ε}-bis-(tert-Butoxycarbonyl)-L-lysin, 18mg N-Hydroxybenzotriazol und 20mg N-Ethyl-N'-(3-dimethylaminopropyl)-carbodiimid Hydrochlorid in 5ml DMF gegeben und zwei Stunden bei Raumtemperatur gerührt. Man engt ein und verrührt den Rückstand mit Wasser. Anschließend wird das erhaltene Produkt in 5ml Dichlormethan aufgenommen, bei 0°C mit 500µml Trifluoressigsäure versetzt und eine Stunde bei Raumtemp. gerührt. Nach Einengen verrührt man den Rückstand mit Ether und fällt das Produkt aus Dichlormethan/Ether. Die Zielverbindung wird in 52%iger Gesamtausbeute erhalten. [DC: Acetonitril/Wasser/Eisessig 10:5:3 R_{f} = 0,15]

### 3.1) 10,11-(Ethylendioxy)-7-{1-[(N^{α},N^{ε}-bis-(O-(3-O-methyl-β-L-fucopyranosyl)-4-yl-hydroxy-phenylamino-thiocarbonyl)-L-lysyl-L-alanyl]-piperazin-4-yl-methyl}-20(R/S)-camptothecin

In Analogie zu Beispiel 1.1 wird durch Kupplung des Peptidkonjugats 3.1.d mit dem Kohlenhydratderivat aus Beispiel I.1 das gewünschte Glycokonjugat hergestellt. Ausb.: 77% [DC: Acetonitril/Wasser 10:1 R_{f} = 0,31] [FAB-MS: m/z = 1326 = M+H].

### Beispiel 3.2:

### 10,11-(Ethylendioxy)-7-{1-[(N^{α},N^{ε}-bis-(O-(3-O-methyl-β-L-fucopyranosyl)-4-yl-hydroxy-phenylamino-thiocarbonyl)-L-lysyl-L-alanyl]-piperazin-4yl-methyl}-20(S)-camptothecin

Dieses Produkt wird in völliger Analogie zu dem 20-(R/S)-Gemisch in Beispiel 3 1 aus dem enantiomerenreinen S-konfigurierten Tricyclus hergestellt.

### Beispiel 3.3:

### 10,11-(Ethylendioxy)-7-{1-[N^{α}-(O-(3-O-carboxymethyl-β-L-fucopyranosyl)-4-hydroxy-phenylamino-thiocarbonyl]-L-lysyl-L-alanyl]-piperazin-4-yl-methyl}-20(R/S)-camptothecin, Hydrochlorid

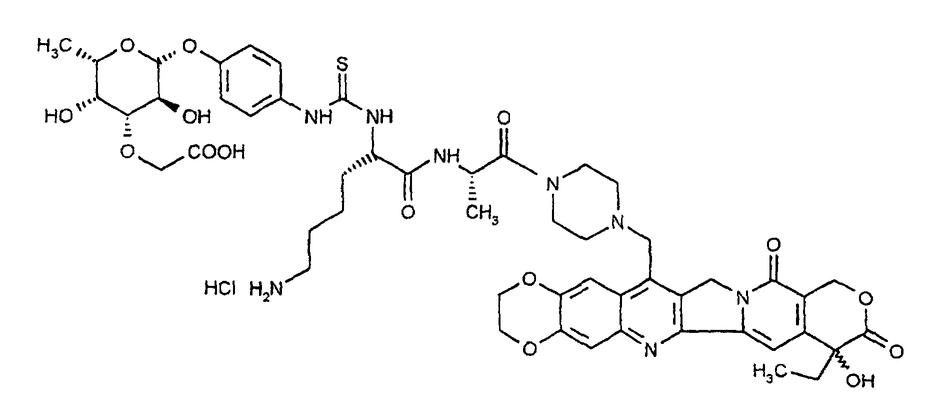

Dieses Konjugat wird in Analogie zu den Beispielen 1.2.a, 1.2.b und 1.2 aus dem L-Alanyl-Konjugat in Beispiel 3.1.c hergestellt.

### Beispiel 3.4:

### 10,11-(Ethylendioxy)-7-{1-[N^{α}-(O-(3-O-carboxymethyl-β-L-fucopyranosyl)-4-hydroxy-phenylamino-thiocarbonyl]-L-lysyl-L-valyl]-piperazin-4-yl-methyl}-20(R/S)-camptothecin, Hydrochlorid

Die Verbindung wird analog zu Beispiel 3.3 hergestellt.

### Beispiel 3.5:

### 10,11-(Ethylendioxy)-7-{1-[N^{α}-(O-(3-O-methyl-β-L-fucopyranosyl)-4-hydroxyphenylamino-thiocarbonyl]-L-lysyl-L-alanyl]-piperazin-4-yl-methyl}-20(R/S)-camptothecin, Hydrochlorid

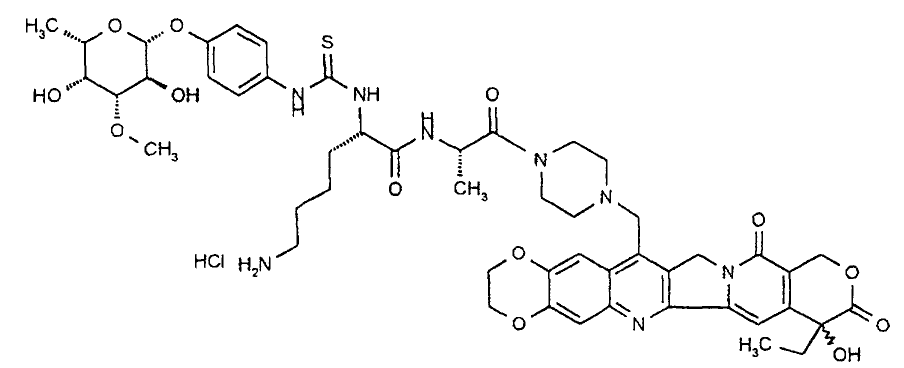

Dieses Konjugat wird in Analogie zu Beispiel 3.3 mit dem Kohlenhydrat aus Beipiel I.1 hergestellt. [DC: Acetonitril/Wasser/Eisessig 10:3:1,5 R_{f} = 0,48]

### Beispiel 4.1:

### 10,11-(Ethylendioxy)-7-{[N^{α}-(O-(3-O-methyl-β-L-fucopyranosyl)-4-hydroxyphenylamino-thiocarbonyl]-L-lysyl-L-alanyl]-aminomethyl}-20(S)-camptothecin, Hydrochlorid

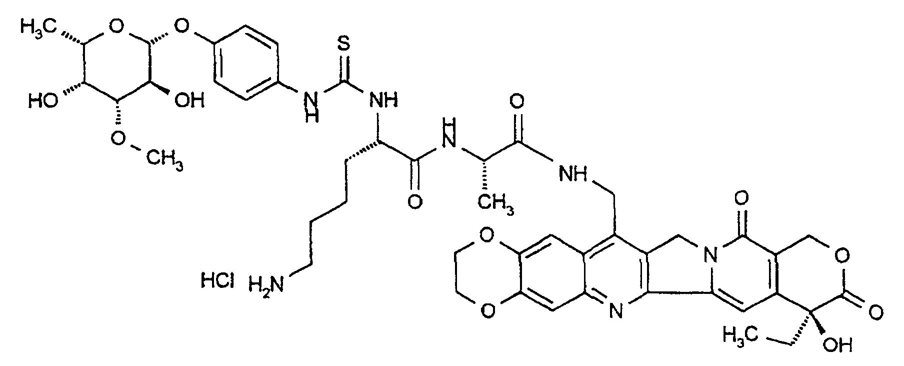

### 4.1.a) 10,11-(Ethylendioxy)-7-aminomethyl-20(S)-camptothecin

Diese Verbindung wird nach den Vorschriften von Luzzio et al. (EP 540099) hergestellt. Ausb.: 32% [DC: Dichlormethan/Methanol 5:1 R_{f} = 0,33]

### 4.1.b) 10,11-(Ethylendioxy)-7-[L-alanyl-aminomethyl]-(20S)-camptothecin

N-(tert.Butoxycarbonyl)-L-alanin-N-hydroxysuccinimidester (0,275mmol) werden in 10ml Dimethylformamid mit 0,275mmol der Verbindung aus Beispiel 4.1 a und mit 0,55mmol Ethyl-diisopropylamin versetzt. Nach 2h Rühren bei Raumtemp engt man ein und fällt den Rückstand aus Dichlormethan/Methanol (1:1) mit Ether. Nach wiederholter Fällung erhält man die Boc-geschützte Zwischenstufe in 70 %iger Ausbeute. Das Produkt wird in 10ml Dichlormethan aufgenommen und zur Ablösung der Boc-Schutzgruppe mit 2ml wasserfreier Trifluoressigsäure versetzt. Nach 30min Rühren bei Raumtemp. engt man ein und fällt die Zielverbindung aus Dichlormethan/Methanol mit Ether. Der Rückstand wird in Wasser aufgenommen und mit Dichlormethan ausgeschüttelt. Nach Einengen der wäßrigen Phase wird das Produkt erneut aus Dichlormethan/Methanol mit Ether gefällt. Ausb.: 69% [DC: Acetonitril/Wasser/Eisessig 5:1:0,2 R_{f} = 0,25]
[FAB-MS: m/z = 507 = M+H].

### 4.1.c) 10,1 1 -(Ethylendioxy)-7-[N^{ε}-(fluorenyl-9-methoxyearbonyl)-L-lysyl-L-alanyl-aminomethyl]-camptothecin, Trifluoracetat:

Das Konjugat aus Beispiel 4.1.b wird nach Standard-Vorschrift mit N^{α}-(tert-Butoxycarbonyl)-N^{ε}-(fluorenyl-9-methoxycarbonyl)-L-lysin verknüpft und anschließend an der α-Aminofunktion deblockiert.

### 4.1.d) 10,11-(Ethylendioxy)-7-{[N^{α}-(O-(3-O-methyl-β-L-fucopyranosyl)-4-hydroxy-phenylamino-thiocarbonyl]-N^{ε}-[fluorenyl-9-methoxycarbonyl]-L-lysyl-L-alanyl-aminomethyl}-20(S)-camptothecin

Die Verbindung aus Beispiel 4.1.c wird in Analogie zu Beispiel 1.1 mit 1,2 Äquivalenten des Kohlenhydratderivats aus Beispiel I.1 umgesetzt. Ausb.: 83%.

### 4.1) 10,11-(Ethylendioxy)-7-{[N^{α}-(O-(3-O-methyl-β-L-fucopyranosyl)-4-hydroxy-phenylamino-thiocarbonyl]-L-lysyl-L-alanyl-aminomethyl}-20(S)-camptothecin, Hydrochlorid

Das Konjugat 4.1.d wird mit Piperidin in DMF deblockiert. Nach 30min. engt man ein und fällt das Produkt zweimal aus Dichlormethan/Methanol mit Ether. Anschließend nimmt man in Wasser auf, versetzt mit 1 Äquivalent einer 0,01N HCI-Lösung und lyophilisiert. Ausb.: 69% [DC: Acetonitril/Wasser/Eisessig 10:3:1,5 R_{f} = 0,39][ESI-MS: m/z = 946 = M+H].

## Patentansprüche

1. Verbindungen der allgemeinen Formel (I) 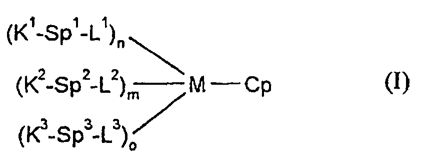 worin
n, m und o jeweils für die Zahl 0 oder 1 stehen und n + m + o ≥1 gilt,
wobei
Cp für ein Camptothecin-Derivat der Formeln 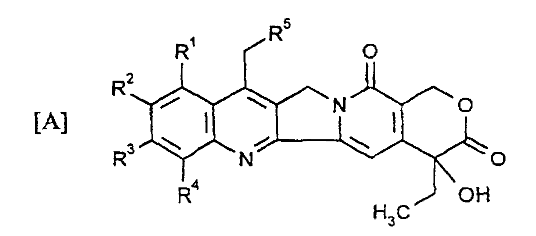 worin
R¹, R², R³ und R⁴ unabhängig voneinander Wasserstoff, Alkyl mit bis zu 3 Kohlenstoffatomen, Halogen, Amino, Hydroxy oder Nitro bedeuten können oder
R² und R³ zusammen eine Gruppe der Formel 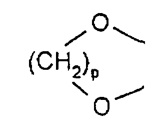 bedeuten, wobei
p die Werte 1 oder 2 annehmen kann, und
R⁵ für -O-* , -NH* oder 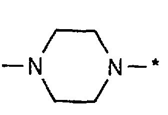 steht,
oder für -*NR⁶ steht, worin R⁶ Arylmethyl oder Hetarylmethyl bedeutet, 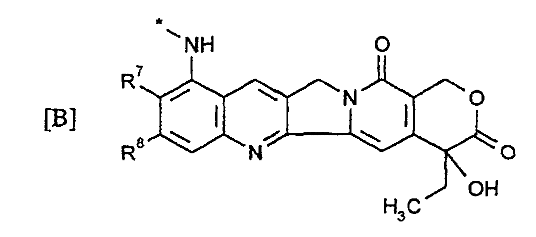 worin
R⁷ und R⁸ wie R² und R³ definiert sind und mit diesen gleich oder verschieden sein können, 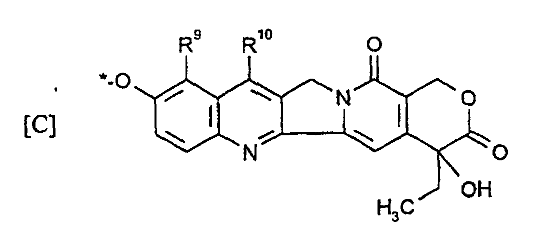 worin
R⁹ für Wasserstoff oder -CH₂-N(CH₃)₂ steht und
R¹⁰ für Wasserstoff oder Ethyl steht, 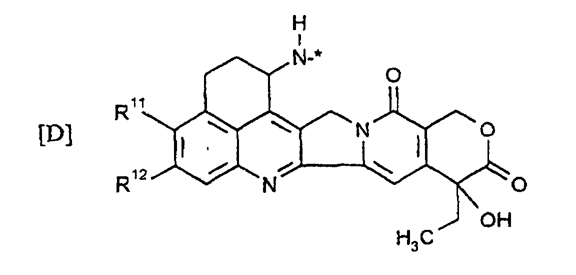 worin
R¹¹ und R¹² wie R² und R³ definiert sind und mit diesen gleichen oder verschieden sein können,
oder 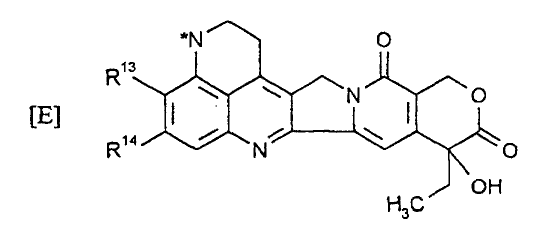 worin
R¹³ und R¹⁴ wie R² und R³ definiert sind und mit diesen gleich oder verschieden sein können,
steht, wobei Cp über die mit * markierten Bindungen mit M verknüpft ist,
M für eine Brücken-Gruppierung steht, deren Hauptkette bis zu 21 Atome in linearer Abfolge umfaßt,
L¹, L², L³ unabhängig voneinander für Linker-Gruppierungen stehen, wie sie in der Glycokonjugat-Chemie gängigerweise eingesetzt werden,
Sp¹, Sp² und Sp³ unabhängig voneinander für Arylen mit bis zu 10 Kohlenstoffatomen oder für Alkylen mit bis zu 8 Kohlenstoffatomen stehen die jeweils gegebenenfalls substituiert sind, und
K¹, K² und K³ unabhängig voneinander für einen Rest der Formel (II) 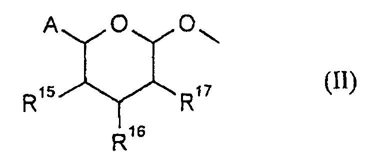 stehen, worin
A Methyl, Hydroxymethyl, Alkoxymethyl mit bis zu 6 Kohlenstoffatomen, Acyloxymethyl mit bis zu 6 Kohlenstoffatomen oder einen Rest der Formel -CH₂-B bedeutet, worin
B für einen Rest der Formel (II) steht,
R¹⁵, R¹⁶ und R¹⁷ unabhängig voneinander Wasserstoff, Hydroxy, gegebenenfalls durch Hydroxyl substituiertes Alkoxy mit bis zu 6 Kohlenstoffatomen, gegebenenfalls durch Alkyl oder Acyl mit bis zu 6 Kohlenstoffatomen substituiertes Amino, Halogen, Sulfat oder eine Gruppe der Formeln 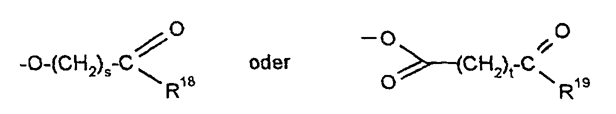 bedeuten, worin
R¹⁸ und R¹⁹ unabhängig voneinander für Hydroxyl oder Alkoxy mit bis zu 6 Kohlenstoffatomen stehen oder für Amino, das gegebenenfalls durch Alkyl mit bis zu 6 Kohlenstoffatomen substituiert ist, stehen und
s und t unabhängig voneinander die Werte 0, 1, 2, 3 oder 4 annehmen können
oder
R¹⁵, R¹⁶ und R¹⁷ unabhängig voneinander für einen Rest der Formel (II) stehen
oder
zwei der Reste R¹⁵, R¹⁶, R¹⁷ zusammen für eine Epoxygruppe stehen,
sowie deren Isomere, Isomerengemische und Salze.

2. Verbindungen der allgemeinen Formel (I) gemäß Anspruch 1, worin K¹, K² und K³ unabhängig voneinander für einen Rest der Formel (II) stehen können, wobei
A Methyl, Hydroxymethyl, Methoxymethyl oder Acetoxymethyl bedeutet,
R¹⁵ Wasserstoff, Hydroxyl, Methoxy oder eine Gruppe der Formeln 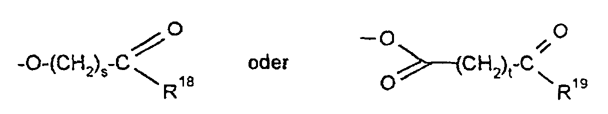 bedeutet, worin
s und t unabhängig voneinander die Werte 1 oder 2 annehmen können und
R¹⁸ und R¹⁹ unabhängig voneinander für Hydroxyl oder Alkoxy mit bis zu 4 Kohlenstoffatomen stehen,
oder
R¹⁵ für einen Rest der Formel (II) steht,
R¹⁶ Wasserstoff, Hydroxyl, Halogen, Alkoxy mit bis zu 4 Kohlenstoffatomen, Sulfat oder eine Gruppe der Formeln 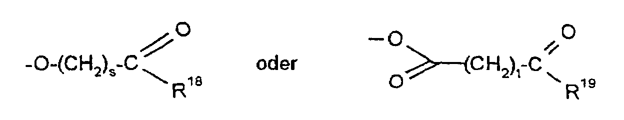 bedeutet, worin
s und t unabhängig voneinander die Werte 1 oder 2 annehmen können und
R¹⁸ und R¹⁹ unabhängig voneinander für Hydroxyl oder Alkoxy mit bis zu 4 Kohlenstoffatomen stehen oder für Amino, das gegebenenfalls durch Alkyl mit bis zu 4 Kohlenstoffatomen substituiert ist, stehen,
R¹⁷ Hydroxyl, Alkoxy mit bis zu 4 Kohlenstoffatomen, das gegebenenfalls durch Hydroxy substituiert ist, gegebenenfalls durch Alkyl oder Acyl mit bis zu 4 Kohlenstoffatomen substituiertes Amino oder eine Gruppe der Formeln 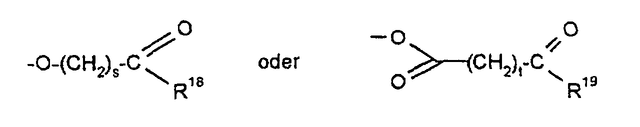 bedeutet, worin
s und t unabhängig voneinander die Werte 1 oder 2 annehmen können und
R¹⁸ und R¹⁹ unabhängig voneinander für Hydroxyl oder Alkoxy mit bis zu 4 Kohlenstoffatomen stehen,
oder worin
R¹⁵ und R¹⁶ gemeinsam für eine Epoxygruppe stehen,
sowie deren Isomere, Isomerengemische und Salze.

3. Verbindungen der allgemeinen Formel (I) gemäß Anspruch 1, worin
Sp¹, Sp² und Sp³ unabhängig voneinander für Arylen mit bis zu 10 Kohlenstoffatomen stehen können, das mit je einer Gruppe K¹ bzw. K² oder K³ und L¹ bzw. L² oder L³ verknüpft ist und das gegebenenfalls noch ein- oder mehrfach durch Hydroxy, Carboxy, Carboxyalkyl mit bis zu 4 Kohlenstoffatomen, Nitro, Cyano, Halogen, Alkyl mit bis zu 4 Kohlenstoffatomen, Halogenalkyl mit bis zu 4 Kohlenstoffatomen, Alkoxy mit bis zu 4 Kohlenstoffatomen substituiert ist,
sowie deren Isomere, Isomerengemische und Salze

4. Verbindungen der allgemeinen Formel (I) gemäß Anspruch 1, worin
L¹, L² und L³ unabhängig voneinander 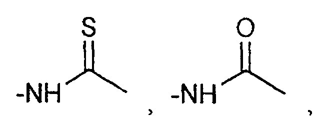 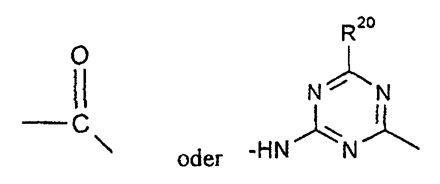 bedeuten, wobei
R²⁰ für Chlor oder für Hydroxyalkylamino mit bis zu 6 Kohlenstoffatomen steht,
sowie deren Isomere, Isomerengemische und Salze.

5. Verbindungen der allgemeinen Formel (I) gemäß Anspruch 1, worin
M für ein Peptid steht, das über eine Aminofunktion mit L¹, L² und/oder L³ verknüpft ist, über eine Acylfunktion mit Cp verknüpft ist und dessen Aminosäurebausteine gegebenenfalls Schutzgruppen tragen können,
sowie deren Isomere, Isomerengemische und Salze.

6. Verbindungen gemäß Anspruch 5, **dadurch gekennzeichnet, daß** M für ein Mono-, Di- oder Tripeptid steht.

7. Verbindungen gemäß Anspruch 5 oder 6, **dadurch gekennzeichnet, daß** das Peptid aus gegebenenfalls Schutzgruppen tragenden Aminosäurebausteinen besteht, ausgewählt aus der Gruppe, bestehend aus Glycyl, Alanyl, Valyl, Leucyl, Lysyl, Seryl, Glutamyl, Threonyl, Asparagyl, Isoleucyl, Diaminopropionyl, Diaminobutyryl, Histidyl, Arginyl und/oder Ornithyl.

8. Verfahren zur Herstellung von Verbindungen der allgemeinen Formel (I) gemäß Anspruch 1, **dadurch gekennzeichnet, daß** man Verbindungen der allgemeinen Formel (III)
Cp-H (III)
worin Cp die in Anspruch 1 Bedeutung hat und das Wasserstoffatom an den mit * bezeichneten Positionen sitzt, mit einer dem in Anspruch 1 Rest M entsprechenden aktivierten Carboxylkomponente Ma, die gegebenenfalls Schutzgruppen trägt, in einem geeigneten Lösungsmittel, gegebenenfalls in Gegenwart einer Base, nach üblichen Methoden umsetzt, gegebenenfalls mittels bekannter Methoden selektiv eine, mehrere oder alle Schutzgruppen von M abspaltet und das Produkt mit Verbindungen der allgemeinen Formel (IV)
K¹-Sp¹-L¹a (IV)
worin K¹ und Sp¹ wie in Anspruch 1 sind und L¹a für eine reaktive Vorstufe der Gruppe L¹ steht, umsetzt, wobei gegebenenfalls die Schutzgruppen selektiv abgespalten und schrittweise verschiedene Gruppen K²-Sp²-L²- und K³-Sp³-L³- in vergleichbarer Weise eingeführt werden können
oder daß man, falls M ein Peptid bedeutet, in vergleichbarer Weise nach üblichen Methoden einen ersten Aminosäurerest in Form der entsprechenden gegebenenfalls Schutzgruppen tragenden aktivierten Carboxylkomponente einführt, gegebenenfalls Schutzgruppen abspaltet und weiterhin gegebenenfalls Schutzgruppen tragende Aminosäurereste anknüpft, wie oben angegeben Reste der Formeln K¹-Sp¹-L¹, K²-Sp²-L² und/oder K³-Sp³-L³-einführt und falls erforderlich Schutzgruppen abspaltet,
daß man weiterhin gegebenenfalls nach üblichen Methoden die Stereoisomeren trennt und daß man gegebenenfalls die Verbindungen in ihre Salze überführt,

9. Verwendung der Verbindungen der allgemeinen Formel (I) gemäß Anspruch 1 zur Herstellung von Arzneimitteln.

10. Arzneimittel enthaltend Verbindungen der allgemeinen Formel (I) gemäß Anspruch 1.

## Claims

1. Compounds of the general formula (I) 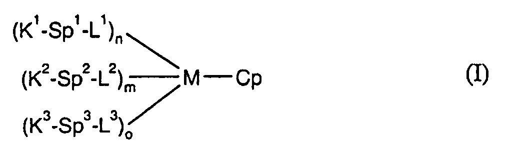 in which
n, m and o in each case represent the number 0 or 1 and n + m + 0 is ≥ 1
where
Cp represents a camptothecin derivative of the formulae 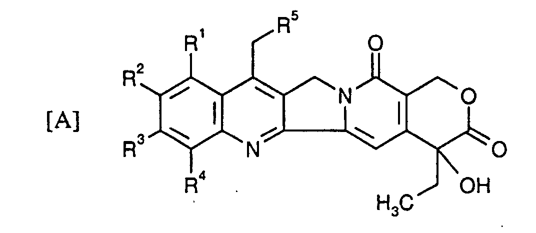 in which
R¹, R², R³ and R⁴ independently of one another may represent hydrogen, alkyl having up to 3 carbon atoms, halogen, amino, hydroxyl or nitro or
R² and R³ together represent a group of the formula 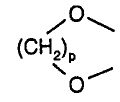 where
P may have the values 1 or 2 and
R⁵ represents -O-*, -NH* or 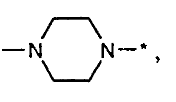 or represents -*NR⁶, in which R⁶ represents arylmethyl or hetarylmethyl, 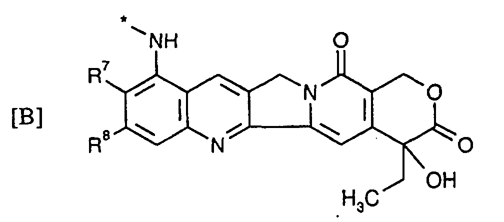
in which
R⁷ and R⁸ are as defined for R² and R³ and may be identical or different to these, 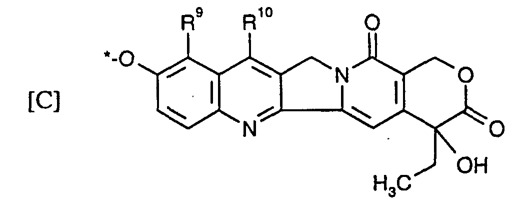 in which
R⁹ represents hydrogen or -CH₂-N(CH₃)₂ and
R¹⁰ represents hydrogen or ethyl, 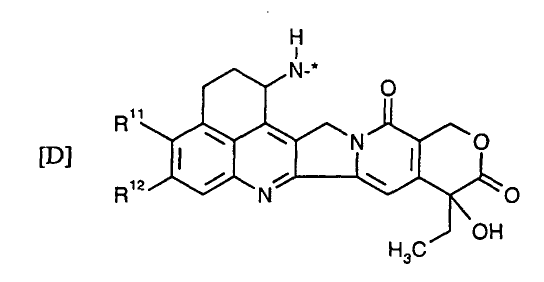 in which
R¹¹ and R¹² are as defined for R² and R³ and may be identical or different to these,
or 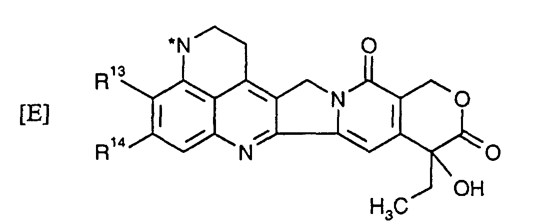 in which
R¹³ and R¹⁴ are as defined for R² and R³ and may be identical or different to these,
where Cp is attached to M via the bonds labelled *,
M represents a bridge grouping whose main chain includes up to 21 atoms in linear order,
L¹, L² and L³ independently of one another each represent linker groupings customarily used in glycoconjugate chemistry,
Sp¹, Sp² and Sp³ independently of one another each represent arylene having up to 10 carbon atoms or represent alkylene having up to 8 carbon atoms which are in each case optionally substituted, and
K¹, K² and K³ independently of one another each represent a radical of the formula (II) 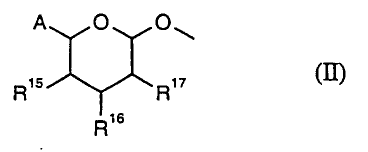 in which
A represents methyl, hydroxymethyl, alkoxymethyl having up to 6 carbon atoms, acyloxymethyl having up to 6 carbon atoms or a radical of the formula -CH₂-B in which
B represents a radical of the formula (II),
R¹⁵, R¹⁶ and R¹⁷ independently of one another each represent hydrogen, hydroxyl, optionally hydroxyl-substituted alkoxy having up to 6 carbon atoms, amino which is optionally substituted by alkyl or acyl having up to 6 carbon atoms, halogen, sulphate or a group of the formula 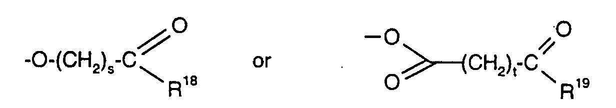 in which
R¹⁸ and R¹⁹ independently of one another each represent hydroxyl or alkoxy having up to 6 carbon atoms or represent amino which is optionally substituted by alkyl having up to 6 carbon atoms, and
s and t independently of one another may each have the values 0, 1, 2, 3 or 4,
or
R¹⁵, R¹⁶ and R¹⁷ independently of one another each represent a radical of the formula (II)
or
two of the radicals R¹⁵, R¹⁶, R¹⁷ together represent an epoxy group,
and their isomers, isomer mixtures and salts.

2. Compounds of the general formula (I) according to Claim 1, in which K¹, K² and K³ independently of one another may each represent a radical of the formula (II) where
A represents methyl, hydroxymethyl, methoxymethyl or acetoxymethyl,
R¹⁵ represents hydrogen, hydroxyl, methoxy or a group of the formula 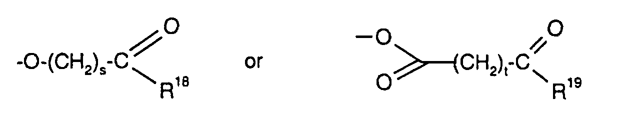 in which
s and t independently of one another may each have the values 1 or 2 and
R¹⁸ and R¹⁹ independently of one another each represent hydroxyl or alkoxy having up to 4 carbon atoms,
or
R¹⁵ represents a radical of the formula (II),
R¹⁶ represents hydrogen, hydroxyl, halogen, alkoxy having up to 4 carbon atoms, sulphate or a group of the formula 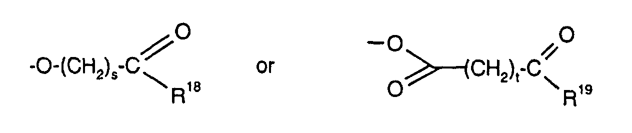 in which
s and t independently of one another may each have the values 1 or 2 and
R¹⁸ and R¹⁹ independently of one another each represent hydroxyl or alkoxy having up to 4 carbon atoms or represent amino which is optionally substituted by alkyl having up to 4 carbon atoms,
R¹⁷ represents hydroxyl, alkoxy having up to 4 carbon atoms which is optionally substituted by hydroxyl, amino which is optionally substituted by alkyl or acyl having up to 4 carbon atoms, or a group of the formula 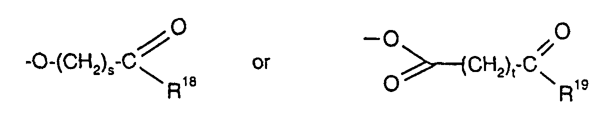 in which
s and t independently of one another may each have the values 1 or 2 and
R¹⁸ and R¹⁹ independently of one another each represent hydroxyl or alkoxy having up to 4 carbon atoms,
or in which
R¹⁵ and R¹⁶ ' together represent an epoxy group,
and their isomers, isomer mixtures and salts.

3. Compounds of the general formula (I) according to Claim 1 in which
Sp¹, Sp² and Sp³ independently of one another may each represent arylene having up to 10 carbon atoms which is attached to in each case one group K¹ and/or K² or K³ and L¹ and/or L² or L³ and which is optionally also mono- or polysubstituted by hydroxyl, carboxyl, carboxyalkyl having up to 4 carbon atoms, nitro, cyano, halogen, alkyl having up to 4 carbon atoms, halogenoalkyl having up to 4 carbon atoms, alkoxy having up to 4 carbon atoms,
and their isomers, isomer mixtures and salts.

4. Compounds of the general formula (I) according to Claim 1 in which
L¹, L² and L³ independently of one another each represent 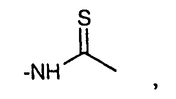 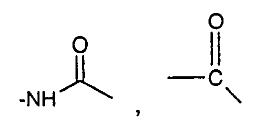 or 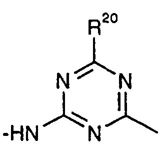 where
R²⁰ represents chlorine or represents hydroxyalkylamino having up to 6 carbon atoms,
and their isomers, isomer mixtures and salts.

5. Compounds of the general formula (I) according to Claim 1 in which
M represents a peptide which is attached to L¹, L² and/or L³ via an amino function, is attached to Cp via an acyl function and whose amino acid building blocks may optionally carry protective groups,
and their isomers, isomer mixtures and salts.

6. Compounds according to Claim 5, **characterized in that** M represents a mono-, di- or tripeptide.

7. Compounds according to Claim 5 or 6, **characterized in that** the peptide comprises amino acid building blocks selected from the group consisting of glycyl, alanyl, valyl, leucyl, lysyl, seryl, glutamyl, threonyl, asparagyl, isoleucyl, diaminopropionyl, diaminobutyryl, histidyl, arginyl and/or ornithyl which optionally carry protective groups.

8. Process for preparing compounds of the general formula (I) according to Claim 1, **characterized in that** compounds of the general formula (III)
Cp-H (III)
in which Cp is as defined in Claim 1 and the hydrogen atom is located on the positions labelled *, are reacted with an activated carboxyl component Ma which corresponds to the radical M defined in Claim 1 and optionally carries protective groups, in a suitable solvent, if appropriate in the presence of a base, by customary methods, one, more than one or all protective groups of M are, if appropriate, selectively removed by known methods and the product is reacted with compounds of the general formula (IV)
K¹-Sp¹-L¹a (IV)
in which K¹ and Sp¹ are each as defined in Claim 1 and L¹a represents a reactive precursor of the group L¹, where the protective groups are, if appropriate, selectively removed and various groups K2-Sp2-L2- and K³-Sp³-L³- can be introduced stepwise in a comparable manner
or that, if M is a peptide, a first amino acid radical is introduced in a comparable manner by customary methods in the form of a corresponding activated carboxyl component which optionally carries protective groups, protective groups are, if appropriate, removed and amino acid radicals which optionally carry protective groups are furthermore attached, radicals of the formulae K¹-Sp¹-L¹-, K²-Sp²-L²- and/or K³-Sp³-L³- are introduced as stated above and, if required, protective groups are removed,
that furthermore, if required, the stereoisomers are separated by customary methods and that the compounds are, if required, converted into their salts.

9. Use of compounds of the general formula (I) according to Claim 1 for preparing medicaments.

10. Medicaments, comprising compounds of the general formula (I) according to Claim 1.

## Revendications

1. Composés de formule générale (I) 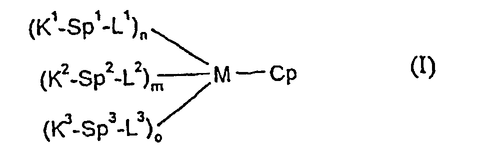 dans laquelle
n, m et o représentent chacun le nombre 0 ou 1 et la somme
n + m + o est supérieure ou égale à 1,
Cp désigne un dérivé de camptothécine de formules 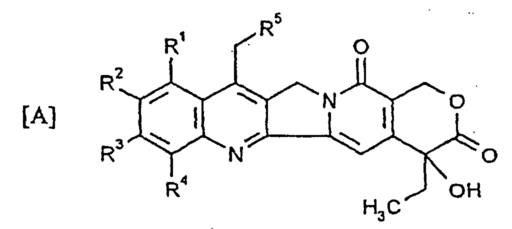 dans laquelle
R¹, R², R³ et R⁴ peuvent représenter, indépendamment les uns des autres, l'hydrogène, un groupe alkyle ayant jusqu'à 3 atomes de carbone, un halogène, un groupe amino, hydroxy ou nitro, ou bien
R² et R³ forment ensemble un groupe de formule 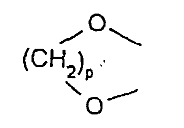
p peut prendre les valeurs 1 ou 2, et
R⁵ représente -O-*, -NH* ou 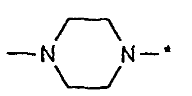 ou bien un groupe -*NR⁶ dans lequel R⁶ est un groupe arylméthyle ou hétarylméthyle, 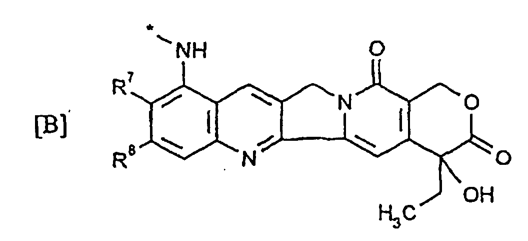 dans laquelle
R⁷ et R⁸ ont les mêmes définitions que R² et R³ et peuvent y être identiques ou en être différents, 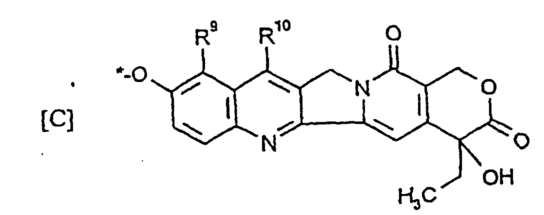 dans laquelle
R⁹ représente l'hydrogène ou un groupe -CH₂-N(CH₃)₂ et
R¹⁰ représente l'hydrogène ou un groupe éthyle, 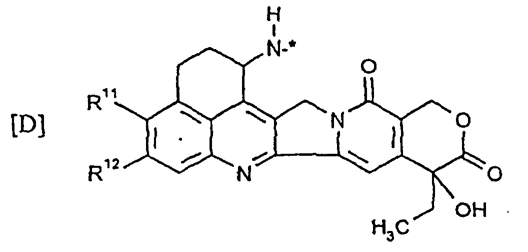 dans laquelle
R¹¹ et R¹² ont la même définition que R² et R³ et peuvent y être identiques ou en être différents,
ou 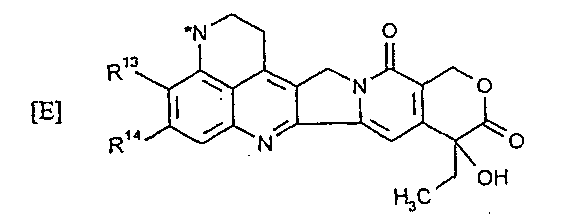 dans laquelle
R¹³ et R¹⁴ ont les mêmes définitions que R² et R³ et peuvent y être identiques ou en être différents,
Cp étant lié à M par l'intermédiaire des liaisons marquées d'un signe *,
M représente un groupement de pontage dont la chaîne principale comprend jusqu'à 21 atomes en succession linéaire,
L¹, L², L³ représentent indépendamment les uns des autres des groupements de liaison tels qu'on en utilise couramment dans la chimie des glyco-conjugués,
Sp¹, Sp² et Sp³ représentent indépendamment les uns des autres des groupes arylène ayant jusqu'à 10 atomes de carbone ou des groupes alkylène ayant jusqu'à 8 atomes de carbone, chacun étant éventuellement substitué, et éventuellement substitué, et
K¹, K² et K³ représentent indépendamment les uns des autres un reste de formule (II) 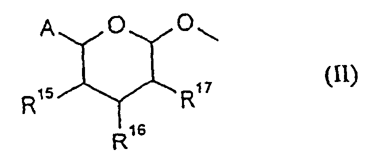 dans laquelle
A est un groupe méthyle, hydroxyméthyle, alkoxyméthyle ayant jusqu'à 6 atomes de carbone, acyloxyméthyle ayant jusqu'à 6 atomes de carbone ou un reste de formule -CH₂-B, dans laquelle
B est un reste de formule (II),
R¹⁵, R¹⁶ et R¹⁷ représentent indépendamment les uns des autres l'hydrogène, un groupe hydroxy, un groupe alkoxy ayant jusqu'à 6 atomes de carbone portant éventuellement un substituant hydroxyle, un groupe amino éventuellement substitué par un radical alkyle ou acyle ayant jusqu'à 6 atomes de carbone, un halogène, un groupe sulfate ou un groupe de formules 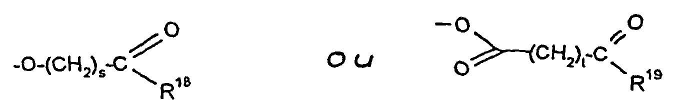 dans lesquelles
R¹⁸ et R¹⁹ représentent indépendamment l'un de l'autre un groupe hydroxyle ou un groupe alkoxy ayant jusqu'à 6 atomes de carbone, ou un groupe amino qui est éventuellement substitué par un radical alkyle ayant jusqu'à 6 atomes de carbone, et
s et t peuvent prendre indépendamment l'un de l'autre les valeurs 0, 1, 2, 3 ou 4
ou bien
R¹⁵, R¹⁶ et R¹⁷ représentent indépendamment les uns des autres un reste de formule (II)
ou bien
deux des restes R¹⁵, R¹⁶, R¹⁷ forment ensemble un groupe époxy,
ainsi que leurs isomères, leurs mélanges d'isomères et leurs sels.

2. Composés de formule générale (I) suivant la revendication 1, dans laquelle K¹, K² et K³ peuvent représenter indépendamment les uns des autres un reste de formule (II),
où
A représente un groupe méthyle, hydroxyméthyle, méthoxyméthyle ou acétoxyméthyle,
R¹⁵ est l'hydrogène, un groupe hydroxyle, méthoxy ou un groupe de formules 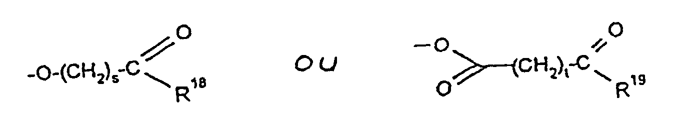 dans lesquelles
s et t peuvent prendre indépendamment l'un de l'autre les valeurs 1 ou 2 et
R¹⁸ et R¹⁹ représentent indépendamment l'un de l'autre un groupe hydroxyle ou un groupe alkoxy ayant jusqu'à 4 atomes de carbone,
ou bien
R¹⁵ représente un reste de formule (II),
R¹⁶ représente l'hydrogène, un groupe hydroxyle, un halogène, un groupe alkoxy ayant jusqu'à 4 atomes de carbone, un groupe sulfate ou un groupe de formules 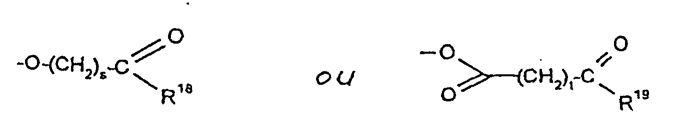 dans lesquelles
s et t peuvent prendre indépendamment l'un de l'autre les valeurs 1 ou 2, et
R¹⁸ et R¹⁹ représentent indépendamment l'un de l'autre un groupe hydroxyle ou un groupe alkoxy ayant jusqu'à 4 atomes de carbone, ou un groupe amino qui est substitué éventuellement par un radical alkyle ayant jusqu'à 4 atomes de carbone,
R¹⁷ représente un groupe hydroxyle, un groupe alkoxy ayant jusqu'à 4 atomes de carbone, qui est substitué le cas échéant par un radical hydroxy, le groupe amino éventuellement substitué par un radical alkyle ou acyle ayant jusqu'à 4 atomes de carbone, ou un groupe de formules 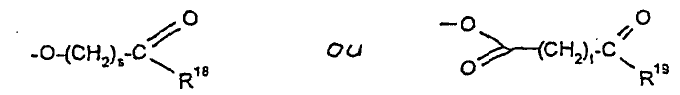 dans lesquelles
s et t peuvent prendre indépendamment l'un de l'autre les valeurs 1 ou 2 et
R¹⁸ et R¹⁹ représentent indépendamment l'un de l'autre un groupe hydroxyle ou un groupe alkoxy ayant jusqu'à 4 atomes de carbone,
ou dans laquelle
R¹⁵ et R¹⁶ représentent indépendamment l'un de l'autre un groupe époxy,
ainsi que leurs isomères, leurs mélanges d'isomères et leurs sels.

3. Composés de formule générale (I) suivant la revendication 1, dans laquelle
Sp¹, Sp² et Sp³ peuvent représenter indépendamment les uns des autres un groupe arylène ayant jusqu'à 10 atomes de carbone, qui est lié dans chaque cas à un groupe K¹ ou respectivement K² ou K³, ou L¹ ou respectivement L² ou L³ et qui est encore substitué le cas échéant une ou plusieurs fois par un radical hydroxy, carboxy, carboxy-alkyle ayant jusqu'à 4 atomes de carbone, nitro, cyano, halogéno, alkyle ayant jusqu'à 4 atomes de carbone, halogénalkyle ayant jusqu'à 4 atomes de carbone, alkoxy ayant jusqu'à 4 atomes de carbone,
ainsi que leurs isomères, leurs mélanges d'isomères et leurs sels.

4. Composés de formule générale (I) suivant la revendication 1, dans laquelle
L¹, L² et L³ représentent indépendamment les uns des autres 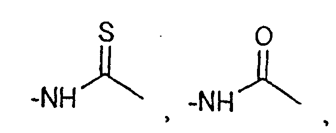 où 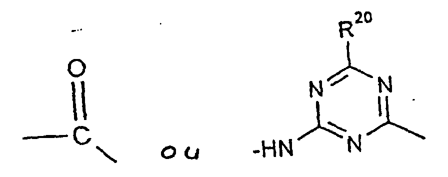
R²⁰ représente le chlore ou un groupe hydroxy-alkylamino ayant jusqu'à 6 atomes de carbone,
ainsi que leurs isomères, leurs mélanges d'isomères et leurs sels.

5. Composés de formule générale (I) suivant la revendication 1, dans laquelle
M désigne un peptide qui est lié à L¹, L² et/ou L³ par l'intermédiaire d'une fonction amino, qui est lié à Cp par l'intermédiaire d'une fonction acyle et dont les éléments amino-acides constitutifs peuvent éventuellement porter des groupes protecteurs,
ainsi que leurs isomères, leurs mélanges d'isomères et leurs sels.

6. Composés suivant la revendication 5, **caractérisés en ce que** M représente un monopeptide, un dipeptide ou un tripeptide.

7. Composés suivant la revendication 5 ou 6, **caractérisés en ce que** le peptide est constitué de motifs amino-acides éventuellement porteurs de groupes protecteurs, ces motifs étant choisis dans le groupe consistant en glycyle, alanyle, valyle, leucyle, lysyle, séryle, glutamyle, thréonyle, asparagyle, isoleucyle, diaminopropionyle, diaminobutyryle, histidyle, arginyle et/ou ornithyle.

8. Procédé de production de composés de formule générale (I) suivant la revendication 1, **caractérisé en ce qu'**on fait réagir selon des modes opératoires classiques des composés de formule générale (III)
Cp-H (III)
dans laquelle Cp a la définition donnée dans la revendication 1, et l'atome d'hydrogène est situé dans les positions marquées d'un signe *, avec un composant carboxyle Ma activé, correspondant au reste M défini dans la revendication 1, qui porte éventuellement des groupes protecteurs, dans un solvant approprié, éventuellement en présence d'une base, on élimine éventuellement par des modes opératoires connus, de façon sélective, une partie ou la totalité des groupes protecteurs de M et on fait réagir le produit avec des composés de formule générale (IV)
K¹-Sp¹-L¹a (IV)
dans laquelle K¹ et Sp¹ sont tels que définis dans la revendication 1, et on peut alors le cas échéant éliminer sélectivement les groupes protecteurs et introduire progressivement de manière comparable divers groupes K²-Sp²-L²- et K³-Sp³-L³-,
ou bien, au cas ou M désigne un peptide, on introduit de manière comparable selon des modes opératoires classiques un premier reste d'amino-acide sous forme du composant carboxylé activé correspondant portant éventuellement des groupes protecteurs, on élimine éventuellement les groupes protecteurs et on rattache en outre des restes d'amino-acides portant éventuellement des groupes protecteurs, on introduit comme indiqué ci-dessus des restes de formules K¹-Sp¹-L¹, K²-Sp²-L² et/ou K³-Sp³-L³ et, si nécessaire, on élimine les groupes protecteurs,
on sépare en outre le cas échéant les stéréo-isomères selon des modes opératoires classiques et on transforme éventuellement les composés en leurs sels.

9. Utilisation des composés de formule générale (I) suivant la revendication 1, pour la préparation de médicaments.

10. Médicaments contenant des composés de formule générale (I) suivant la revendication 1.
